# EUROPÄISCHE PATENTSCHRIFT

(11) **EP 2 688 577 B1**
(45) Veröffentlichungstag und Bekanntmachung des Hinweises auf die Patenterteilung: **23.07.2014**
(21) Anmeldenummer: 12701225.0
(22) Anmeldetag: 24.01.2012
(51) Int. Cl.: A61K 36/534, A61K 36/57, A61K 36/235, A61K 36/23, A61P 1/02

(54) **ZUSAMMENSETZUNG ZUR ANWENDUNG BEI VERDAUUNGSBESCHWERDEN**
DIGESTIVE SYMPTOM AMELIORATING COMPOSITION
COMPOSITION REDUCTRICE DES SYMPTOMES DE TROUBLES DIGESTIFS

(30) Priorität: 25.03.2011 DE 202011004425 U
(43) Veröffentlichungstag der Anmeldung: 29.01.2014
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE)
(74) Vertreter: Von Rohr Patentanwälte Partnerschaft mbB
(86) Internationale Anmeldenummer: PCT/EP2012/000301
(87) Internationale Veröffentlichungsnummer: WO 2012/130351

(56) Entgegenhaltungen:
- WO-A1-2008/056200
- WO-A1-2008/074080
- DE-A1-102008 023 345
- DE-U1-202008 015 430
- US-A1- 2002 114 832

## Beschreibung

Die vorliegende Erfindung betrifft den Bereich der Verdauungsbeschwerden bzw. Verdauungsstörungen und insbesondere den Bereich der dyspeptischen Fehlfunktionen (dyspeptische Beschwerden bzw. Verdauungserkrankungen).

Insbesondere betrifft die vorliegende Erfindung eine Zusammensetzung, insbesondere eine pharmazeutische Zusammensetzung bzw. Zubereitung, welche sich vorzugsweise zur therapeutischen oder prophylaktischen Behandlung von Verdauungsbeschwerden bzw. -störungen, insbesondere Verdauungsfehlfunktionen, eignet. Die Zusammensetzung nach der Erfindung enthält in Kombination mindestens ein Polysiloxan einerseits und eine Kombination spezieller ätherischer Öle andererseits.

Darüber hinaus betrifft die vorliegende Erfindung Dosiereinheiten, welche sich insbesondere zur peroralen Applikation eignen und welche insbesondere in Form einer Kapsel bzw. eines Portionsbeutels vorliegen.

Weiterhin betrifft die vorliegende Erfindung eine Verpackungseinheit, welche mindestens eine Dosiereinheit nach der Erfindung enthält.

Die Zusammensetzung nach der Erfindung eignet sich für die Verwendung zur therapeutischen bzw. prophylaktischen Behandlung von Verdauungsbeschwerden bzw. -störungen sowie für die Verwendung der erfindungsgemäßen Zusammensetzung als Karminativum bzw. zur Herstellung eines Arzneimittels mit karminativer Wirkung.

Unter Verdauungsstörungen versteht man unterschiedlich verursachte Störungen der Verdauungsabläufe mit den Symptomen Appetitlosigkeit, Aufstoßen, Völlegefühl, Magendruck, Blähungen und Durchfall. Nicht organisch bedingte Verdauungsstörungen werden meist als Dyspepsie bezeichnet, während organisch bedingte Fehlfunktionen bzw. Störungen des Verdauungsablaufes als Maldigestionssyndrome bezeichnet werden.

Bei Dyspepsien handelt es sich insbesondere um nicht organisch bedingte Verdauungsstörungen, die dadurch zustande kommen können, dass unverdaute und nicht resorbierte Nahrungsbestandteile in tiefere Darmabschnitte gelangen, wo sie unter Darmreizung zersetzt werden. Abhängig von dem Nahrungsgrundstoff, der unzureichend resorbiert wird, kommt es zu unterschiedlichen Formen der Dyspepsie, nämlich im Falle von Eiweiß zu der so genannten Fäulnis-Dyspepsie, im Falle von Kohlenhydraten zu der so genannten Gährungs-Dyspepsie und schließlich im Falle von Fetten zu der so genannten Seifen-Dyspepsie.

Zudem führt üppiges Essen, Hektik, Stress und vor allem der hohe Anteil an so genannten "versteckten" Fetten in der Nahrung dazu, dass die Verdauung schnell aus dem Gleichgewicht gebracht wird und somit der Magen/Darm-Trakt sowie Leber und Galle belastet werden. Häufig sind Völlegefühl, Blähungen und Magendruck die Folge.

Ein Mangel an Enzymen bzw. Gallenflüssigkeit mit einhergehender ungenügender Spaltung von Nahrungsbestandteilen kann zu Verdauungsstörungen bzw. Maldigestionssyndromen führen. Die Symptome einer Maldigestion umfassen anhaltende Durchfälle, die bei der Störung der Eiweißverdauung durch faulige Zersetzungsprodukte und bei der Störung der Fettverdauung durch den Gehalt an Fettsalzen gekennzeichnet sind.

Funktionsstörungen bzw. Beschwerden der Verdauung können sowohl bei jüngeren als auch bei älteren Personen auftreten. Gerade bei älteren Menschen kann es durch alterungsbedingte Veränderungen an der Darmschleimhaut zu Verdauungsstörungen verschiedenster Art kommen, welche mit den vorgenannten Symptomen einhergehen können.

Zudem ist bei Säuglingen das Störungsbild der so genannten Dreimonatskolik bekannt, welche oftmals mit exzessivem Schreien und Schlaf- sowie Essstörungen der davon betroffenen Säuglinge einhergeht. Hierbei handelt es sich um eine Regulationsstörung im Säuglingsalter, welche auch durch Krankheiten bzw. Fehlfunktionen des Magen/Darm-Traktes bedingt sein kann. Eine gestörte Anpassung der Funktion des kindlichen Magen/Darm-Traktes kann auf unterschiedliche Weise zu krampfartigen Schmerzen beim Säugling führen, wobei in diesem Zusammenhang auch schmerzhafte Auftreibungen des Darms durch Gase eine wichtige Rolle spielen.

Verdauungsbeschwerden bzw. -störungen gehen oftmals mit einer abnormen Luft- bzw. Gasansammlung unterschiedlicher Ursache im Bauchraum einher, wie sie beispielsweise durch eine mangelhafte Verdauung von Nahrungsbestandteilen resultiert, beispielsweise bei übermäßigem Konsum von unverdaulichen Kohlenhydraten, bei Lactoseintoleranz und dergleichen. In diesem Zusammenhang bezeichnet die so genannte Flatulenz die Aufblähung des Magens bzw. des Darms durch bei der Verdauung gebildete Gase (z. B. Methan, Kohlenmonoxid, Kohlendioxid, Schwefelwasserstoff und dergleichen), wobei es häufig zum Entweichen von Darmgasen kommt. Sitzen diese Darmgase fest, kann es zu schmerzhaften Bauchkrämpfen kommen.

Die Flatulenz wird im Allgemeinen von dem so genannten Meteorismus abgegrenzt. Bei Meteorismus handelt es sich um eine übermäßige Gasansammlung im Verdauungstrakt ohne wesentlichen Abgang von Darmgasen, oftmals begleitet von krampfartigen Schmerzen und Koliken. Der Leidensdruck bei den vorgenannten Störungen kann erheblich sein. So ist Meteorismus nicht selten in Verbindung mit Stuhlunregelmäßigkeiten, Schmerzen unterschiedlicher Lokalisation und Ausprägung ein häufiger Anlass zum Aufsuchen des Arztes.

Zur Behandlung der vorgenannten Verdauungsbeschwerden bzw. -störungen ist es im Stand der Technik oftmals vorgesehen, pharmazeutische Präparate mit chemisch-synthetischen Wirkstoffen zur Anwendung zu bringen. Diese Präparate haben oftmals den Nachteil, dass sie von Nebenwirkungen begleitet sind.

Weiterhin kommen Präparate auf Basis natürlicher Wirkstoffe zum Einsatz. Diese Präparate zeigen aber den gewünschten verdauungsfördernden bzw. den die Verdauungsstörungen lindernden Effekt nicht immer in ausreichendem Maße.

So betrifft die DE 20 2008 015 430 U1 eine Zusammensetzung zur Behandlung von Verdauungsbeschwerden oder -störungen, welche in Kombination und jeweils in pharmazeutisch wirksamen Mengen (a) mindestens ein Polysiloxan und (b) mindestens ein Enzym, vorzugsweise ein proteolytisches Enzym bzw. eine Protease, enthält.

Weiterhin betrifft die DE 10 2008 023 345 A1 wasserlösliche, feste, filmförmige Zubereitungen, welche mindestens ein filmbildendes Polymer aus der Gruppe von Polyvinylalkoholen und mindestens eine wasserunlösliche, ölige Flüssigkeit aufweisen, wobei die ölige Flüssigkeit in das filmbildende Polymer eingearbeitet ist. Die ölige Substanz kann unter anderem aus Mineralölen, Syntheseölen, wie Silikonölen, und ätherischen Ölen ausgewählt sei.

Die WO 2008/056200 A1 betrifft orale pharmazeutische Zusammensetzungen, welche Simeticon vorzugsweise in Kombination mit einem zweiten Inhaltsstoff auf Basis von Histamin-Rezeptor Inhibitoren, gastrointestinalen Prokinetika und ähnlichen Substanzen enthalten, und Verfahren zur Herstellung von derartigen Zusammensetzungen.

Die WO 2008/074080 A1 betrifft Zusammensetzungen und Verfahren zur Behandlung von Symptomen des Reizdarmsyndroms, wie Blähungen, Flatulenz oder Schmerzen. Die Zusammensetzungen enthalten eine Kombination therapeutischer Mengen an Pfefferminze, Fenchel und Ingwer und können in Form von Kapseln vorliegen. Zudem können sie zusätzliche Substanzen wie Schafgarbe, Zaubernuss, Schachtelhalm und Aloe Vera-Arten enthalten.

Die US 2002/0114832 A1 betrifft pharmazeutische Zusammensetzungen, welche Pfefferminzöl und Kümmelöl zur Behandlung von Verdauungsbeschwerden und Krämpfen im Gastrointestinaltrakt enthalten. Gemäß besonders bevorzugter Ausführungsformen weisen die Zusammensetzungen Pfefferminzöl und Kümmelöl in besonders hoher Dosierung pro Applikationsform auf.

Insgesamt führen die im Stand der Technik vorgesehenen medikamentösen Ansätze zur Vorbeugung bzw. Behandlung von Verdauungsbeschwerden bzw. -störungen der vorgenannten Art nicht immer zu einem ausreichenden Therapieerfolg und weisen oftmals gravierende Nebenwirkungen auf.

Vor diesem Hintergrund besteht - insbesondere auch aufgrund der großen Verbreitung von Verdauungsbeschwerden oder -störungen - ein großer Bedarf, geeignete Zusammensetzungen, insbesondere pharmazeutische Zusammensetzungen bzw. Zubereitungen, und Therapieformen bereitzustellen, welche sich zur prophylaktischen oder therapeutischen Behandlung der vorgenannten Verdauungsbeschwerden bzw. -störungen eignen.

Somit liegt der vorliegenden Erfindung die Aufgabe zugrunde, eine Zusammensetzung, insbesondere pharmazeutische Zusammensetzung bzw. Zubereitung, bereitzustellen, welche sich zur prophylaktischen oder therapeutischen Behandlung der zuvor genannten Verdauungsbeschwerden bzw. -störungen eignet und welche die zuvor geschilderten Nachteile des Standes der Technik zumindest weitgehend vermeidet oder zumindest abschwächt. Dabei soll eine gute Wirksamkeit bei gleichzeitig hervorragender Verträglichkeit gewährleistet sein.

Eine weitere Aufgabe der vorliegenden Erfindung liegt in der Bereitstellung einer Zusammensetzung, insbesondere in Form einer pharmazeutischen Zubereitung, welche insbesondere eine verdauungsfördernde Wirkung aufweist bzw. zu einer signifikanten Linderung der mit Verdauungsbeschwerden bzw. -störungen einhergehenden Symptome führt. Insbesondere liegt eine Aufgabe der vorliegenden Erfindung darin, eine derartige Zusammensetzung mit verdauungsfördernder Wirkung unter Verwendung von Naturstoffen bzw. Naturstoffpräparaten bereitzustellen.

Die Anmelderin hat nun in völlig überraschender Weise herausgefunden, dass die zuvor geschilderte Aufgabe dadurch gelöst werden kann, dass erfindungsgemäß eine Zusammensetzung bzw. Kombination bzw. Zubereitung bereitgestellt wird, welche mindestens ein Polysiloxan zusammen mit einer sehr speziellen Kombination von ätherischen Ölen, nämlich Pfefferminzöl, Anisöl, Fenchelöl und Kümmelöl, enthält. Denn eine derart spezielle Kombination führt bei entsprechender Anwendung zu einer deutlichen Verringerung von Verdauungsbeschwerden bzw. - störungen und insbesondere zu einer signifikanten Linderung der damit in Verbindung stehenden Symptome bzw. Erkrankungen, wie Meteorismus, Flatulenz, Koliken, Völlegefühl, Magendruck, übermäßiger Gasbildung im Magen/Darm-Trakt und damit einhergehendem Bauch- bzw. Magendruck. Auf Basis der erfindungsgemäßen Zusammensetzung kann die Verdauungsfunktion verbessert werden, wobei auch eine übermäßige Gasbildung im Magen/Darm-Trakt wirksam verringert bzw. unterbunden bzw. die Abfuhr bzw. das Ausscheiden von Gasen aus dem Körper beschleunigt bzw. verbessert wird.

Zur Lösung der zuvor geschilderten Aufgabenstellung schlägt die vorliegende Erfindung somit - gemäß einem ersten Aspekt der vorliegenden Erfindung - eine Zusammensetzung, insbesondere ein Arzneimittel bzw. eine pharmazeutische Zusammensetzung bzw. Zubereitung, gemäß Anspruch 1 vor. Weitere, vorteilhafte Ausgestaltungen der erfindungsgemäßen Zusammensetzung bzw. Zubereitung sind Gegenstand der diesbezüglichen Unteransprüche.

Weiterer Gegenstand der vorliegenden Erfindung - gemäß einem zweiten Aspekt der vorliegenden Erfindung - ist die erfindungsgemäß Dosiereinheit gemäß Anspruch 12, welche insbesondere zur peroralen Applikation geeignet ist und welche die erfindungsgemäße Zusammensetzung aufweist.

Wiederum weiterer Gegenstand der vorliegenden Erfindung - gemäß einem dritten Aspekt der vorliegenden Erfindung - ist die erfindungsgemäße Verpackungseinheit gemäß Anspruch 13, welche mindestens eine Dosiereinheit nach der Erfindung aufweist.

Schließlich ist weiterer Gegenstand der vorliegenden Erfindung - gemäß einem sechsten Aspekt der vorliegenden Erfindung - das Karminativum gemäß Anspruch 14, welches die erfindungsgemäße Zusammensetzung bzw. die erfindungsgemäße Dosiereinheit bzw. die erfindungsgemäße Verpackungseinheit enthält.

Der Begriff "Arzneimittel" - synonym bisweilen auch als "pharmazeutische Zubereitung", "pharmazeutische Zusammensetzung", "pharmazeutische Kombination" etc. bezeichnet - ist im Rahmen der vorliegenden Erfindung sehr breit zu verstehen und umfasst jede Art von möglicher pharmazeutischer Zubereitung, Zusammensetzung oder Kombination, insbesondere Arzneimittel bzw. Pharmaka als solche, aber auch so genannte Medizinprodukte, homöopathische Mittel, Nahrungsergänzungsmittel und dergleichen.

Bei allen nachstehend genannten relativen bzw. prozentualen gewichtsbezogenen Mengenangaben ist zu beachten, dass diese im Rahmen der erfindungsgemäßen Zusammensetzung derart auszuwählen sind, dass sie sich in der Summe in dem jeweiligen Bezugssystem (z. B. in der erfindungsgemäßen Zusammensetzung als solcher) stets um 100 Gew.-% ergänzen. Dies versteht sich für den Fachmann aber von selbst. Im Übrigen gilt, dass der Fachmann anwendungsbezogen oder einzelfallbedingt von den nachfolgend angeführten Mengenangaben abweichen kann, ohne dass er den Rahmen der vorliegenden Erfindung verlässt. Was die Mengenangaben zu den Komponenten (a) bis (e) im Allgemeinen anbelangt, so wird im Rahmen der vorliegenden Erfindung unter der für die Mengenangaben angegebenen Bezugsgröße somit die erfindungsgemäße Zusammensetzung auf Basis der Komponenten (a) bis (e) und ohne Exzipienten bzw. Kapsel- und/oder Hüllmaterialien verstanden.

Es versteht sich zudem von selbst, dass Ausgestaltungen, Ausführungsformen, Vorteile und dergleichen, welche nachfolgend zu Zwecken der Vermeidung von Wiederholungen nur zu einem Erfindungsaspekt angeführt sind, selbstverständlich auch in Bezug auf die übrigen Erfindungsaspekte entsprechend gelten.

Gegenstand der vorliegenden Erfindung - gemäß einem ersten Aspekt der vorliegenden Erfindung - ist somit eine Zusammensetzung, insbesondere pharmazeutische Zubereitung, vorzugsweise zur therapeutischen oder prophylaktischen Behandlung von Verdauungsbeschwerden oder -störungen, insbesondere Verdauungsfehlfunktionen, Meteorismus, Flatulenz, Koliken, Völlegefühl, Magendruck, übermäßiger Gasbildung im Magen/Darm-Trakt oder dergleichen,
wobei die Zusammensetzung - in Kombination und jeweils in wirksamen Mengen, insbesondere pharmazeutisch wirksamen Mengen -
(a) mindestens ein Polysiloxan in einer Menge von 60 bis 99,9 Gew.-%, bezogen auf die Zusammensetzung;
(b) Pfefferminzöl in einer Menge von 0,001 bis 1 Gew.-%, bezogen auf die Zusammensetzung;
(c) Anisöl in Form von Sternanisöl und in einer Menge von 0,0015 bis 1,5 Gew.-%, bezogen auf die Zusammensetzung;
(d) Fenchelöl in Form von Bitterfenchelöl und in einer Menge von 0,002 bis 1,3 Gew.-%, bezogen auf die Zusammensetzung;
(e) Kümmelöl in einer Menge von 0,0005 bis 0,8 Gew.-%, bezogen auf die Zusammensetzung;
enthält, wobei die Zusammensetzung enzymfrei ausgebildet ist.

Insbesondere ist es im Rahmen der vorliegenden Erfindung gelungen, eine Zusammensetzung bereitzustellen, mit welcher Verdauungsbeschwerden wirksam gelindert bzw. verbessert werden können. Im Rahmen der vorliegenden Erfindung wird durch die gezielte Kombination des erfindungsgemäß verwendeten Polysiloxans einerseits mit den sehr speziellen ätherischen Ölen andererseits eine Zusammensetzung bereitgestellt, welche gegenüber den Zusammensetzungen des Standes der Technik - bei gleichzeitig sehr guter Verträglichkeit - eine deutlich verbesserte Wirkung im Hinblick auf die zu behandelnden Verdauungsbeschwerden aufweist.

Dabei ergänzen sich im Rahmen der vorliegenden Erfindung die insbesondere physikalische Wirkung, wie nachfolgend noch beschrieben, des Polysiloxans mit der insbesondere spasmolytisch bzw. krampflösenden und verdauungsfördernden Wirkung der speziell eingesetzten ätherischen Öle in besonderer Weise, so dass eine ausgezeichnete verdauungsfördernde bzw. verdauungsunterstützende Wirkung resultiert und darüber hinaus auch das einhergehende Unwohlsein bzw. die Schmerzsymptomatik verbessert wird.

Dabei ergänzen bzw. verstärken sich die jeweiligen Wirkkomponenten im Rahmen der erfindungsgemäß bereitgestellten Zusammensetzung in einer Weise, welche über die Wirksamkeit der Einzelkomponenten jeweils für sich allein betrachtet hinausgeht, so dass sich die jeweiligen Einzelkomponenten auf Basis ihrer gezielten Kombination in ihrer Wirkung verstärken, was auf das Vorliegen eines synergistischen Effektes der erfindungsgemäß beanspruchten Kombination hindeutet.

Die erfindungsgemäße Zusammensetzung zeichnet sich insbesondere dadurch aus, dass bei ihrer Applikation bzw. Anwendung einer übermäßigen Gasbildung im Magen/Darm-Trakt entgegengewirkt werden kann, und zwar insbesondere insofern, als im Verdauungstrakt entstehendes Gas besser abtransportiert wird bzw. die Entstehung solcher Gase verhindert bzw. vermindert wird. Hierdurch wird in effektiver Weise den vorgenannten Symptomen, wie Meteorismus, Flatulenz, Koliken, Völlegefühl, Magendruck und dergleichen, entgegengewirkt, so dass sowohl dyspeptische Beschwerden als auch Maldigestionssymptome verringert werden können, wobei aufgrund der spasmolytischen bzw. entkrampfenden Wirkung der eingesetzten ätherischen Öle bei Applikation der erfindungsgemäßen Zusammensetzung auch eine verringerte Schmerzsymptomatik bzw. ein gesteigertes Wohlbefinden resultiert.

Bei der erfindungsgemäßen Zusammensetzung handelt es sich insbesondere um ein Karminativum, welches insbesondere bei mit übermäßiger Gasbildung im Magen/Darm-Trakt einhergehenden Erkrankungen bzw. Symptomen, wie Meteorismus, Flatulenz, Koliken und dergleichen, wirksam ist. Die erfindungsgemäße Zusammensetzung eignet sich in diesem Zusammenhang insbesondere zur Linderung bzw. Behandlung von funktionellen Beschwerden des Magen/Darm-Traktes, wie Völlegefühl, vorzeitiges Sättigungsgefühl, Aufstoßen und dergleichen. Zudem kommt eine Anwendung bei gesteigerter Darmgasbildung nach Operationen und zur Vorbereitung von Untersuchungen im Bauchbereich, beispielsweise zur Verringerung des so genannten Gasschattens bei sonographischen Untersuchungen bzw. im Rahmen bzw. zur Vorbereitung von Röntgenuntersuchungen, in Frage. Insbesondere eignet sich die erfindungsgemäße Zusammensetzung auch zur Linderung bzw. Behandlung der so genannten Dreimonatskolik bei Säuglingen.

Was das im Rahmen der vorliegenden Erfindung als Komponente (a) eingesetzte mindestens eine Polysiloxan anbelangt, so handelt es sich hierbei insbesondere um ein Polysiloxan mit oberflächenspannungsmodifizierender und/oder entschäumender Wirkung.

Ohne sich auf diese Theorie festlegen zu wollen, weist das erfindungsgemäß eingesetzte Polysiloxan im Rahmen seiner Anwendung eine physikalische Wirkung auf. Insbesondere beeinflusst das erfindungsgemäß eingesetzte Polysiloxan im Magen/Darm-Trakt die Oberflächenspannung von eingeschlossenen Gasblasen, welche sich als Folge auflösen bzw. zu größeren Gasblasen zusammenfügen, welche dann verbessert resorbiert oder durch Flatulenz ausgeschieden werden können, so dass auf diese Weise im Magen/Darm-Trakt entstehende Gase leichter aus dem Körper abtransportiert bzw. ausgeschieden werden. Dies führt dazu, dass die insbesondere mit der übermäßigen Gasbildung einhergehenden Beschwerden verringert bzw. unterbunden werden. Mit anderen Worten besteht eine zentrale Wirkweise des erfindungsgemäß eingesetzten Polysiloxans darin, dass infolge seiner Einwirkung im Magen/Darm-Trakt vorhandene kleine Gasblasen eines kleinblasigen Schaumes zu größeren Gasblasen zusammengelagert werden, welche in größeren Schüben abtransportiert werden können. Ein weiterer zentraler Vorteil des Einsatzes von Polysiloxan ist darin zu sehen, dass diesbezüglich keine signifikanten Nebenwirkungen vorliegen und die eingesetzte Substanz somit gut verträglich ist.

Erfindungsgemäß ist es von besonderem Vorteil, wenn (a) das Polysiloxan ein vorzugsweise lineares Alkyl- oder Arylpolysiloxan ist. Demnach kann es sich bei dem erfindungsgemäß verwendeten Polysiloxan um ein Alkyl- bzw. Arylsiloxanpolymer handeln.

Erfindungsgemäß kann es gemäß einer besonders bevorzugten Ausführungsform vorgesehen sein, dass (a) das Polysiloxan Simeticon (synonym auch als Simethicon bezeichnet) und/oder Dimeticon (synonym auch als Dimethicon bezeichnet), vorzugsweise Simeticon, ist.

Simeticon, welches chemisch auch als α-(Trimethylsilyl)-ω-methylpoly-[oxy(dimethylsilylen)] bezeichnet wird, stellt ein mit Siliciumdioxid aktiviertes Dimeticon dar. Simeticon weist den Vorteil auf, dass es bei peroraler Applikation nicht vom Körper in den Blutkreislauf aufgenommen wird und somit - neben seiner hervorragenden Wirksamkeit in Bezug auf die Behandlung von Verdauungsfehlfunktionen bzw. -störungen der vorgenannten Art - gut verträglich ist und daher insgesamt als unbedenklich gilt. Für weitere Ausführungen zu dem erfindungsgemäß einsetzbaren Simeticon kann verwiesen werden auf RÖMPP Chemielexikon, 10. Auflage, Band 5, 1998, Stichwort: "Simethicon" sowie auf die dort jeweils in Bezug genommene Literatur, deren gesamter Inhalt hiermit durch Bezugnahme eingeschlossen ist.

Bei dem erfindungsgemäß gleichermaßen einsetzbaren Dimeticon handelt es sich um ein Polydimethylsiloxan. Für diesbezüglich weiterführende Ausführungen kann verwiesen werden auf RÖMPP Chemielexikon, 10. Auflage, Band 2, 1997, Stichwort: "Dimeticon" sowie auf die dort jeweils in Bezug genommene Literatur, deren gesamter Inhalt hiermit durch Bezugnahme eingeschlossen ist.

Was das erfindungsgemäß eingesetzte (a) Polysiloxan anbelangt, so kann dessen Menge in der erfindungsgemäßen Zusammensetzung in weiten Bereichen variieren. Im Allgemeinen enthält die Zusammensetzung nach der Erfindung (a) das mindestens eine Polysiloxan in einer Menge von 60 bis 99,9 Gew.-%, besonders bevorzugt 70 bis 99,85 Gew.-%, bezogen auf die Zusammensetzung.

In diesem Zusammenhang ist im Rahmen der vorliegenden Erfindung unter der als für die Mengenangaben angegebenen Bezugsgröße die erfindungsgemäße Zusammensetzung auf Basis der Komponenten (a) bis (e) und ohne Exzipienten bzw. Kapsel- und/oder Hüllmaterialien zu verstehen.

Was das erfindungsgemäß eingesetzte (a) Polysiloxan weiter anbelangt, so enthält die Zusammensetzung nach der Erfindung das (a) mindestens eine Polysiloxan gleichermaßen in einer Menge von mindestens 60 Gew.-%, besonders bevorzugt mindestens 70 Gew.-%, bezogen auf die Zusammensetzung, enthalten und/oder (a) das mindestens eine Polysiloxan in einer Menge von höchstens 99,99 Gew.-%, insbesondere höchstens 99,98 Gew.-%, vorzugsweise höchstens 99,95 Gew.-%, bevorzugt höchstens 99,9 Gew.-%, besonders bevorzugt höchstens 99,85 Gew.-%, bezogen auf die Zusammensetzung.

Weiterhin kann die Zusammensetzung nach der Erfindung (a) das mindestens eine Polysiloxan je Applikations- und/oder Dosiereinheit in einer Menge von 10 bis 1.000 mg, insbesondere 30 bis 800 mg, vorzugsweise 50 bis 600 mg, bevorzugt 80 bis 400 mg, besonders bevorzugt 100 bis 350 mg, enthalten.

Im Rahmen der vorliegenden Erfindung handelt es sich bei der Applikations- bzw. Dosiereinheit insbesondere um eine für die perorale Applikation geeignete Applikations- bzw. Dosiereinheit, wie eine Kapsel oder dergleichen, insbesondere wie nachfolgend definiert. Gleichermaßen kann es sich bei der Dosiereinheit auch um einen Portionsbeutel handeln, welcher die erfindungsgemäße Zusammensetzung enthält, worauf nachfolgend noch im Detail eingegangen wird.

Erfindungsgemäß kann es zudem vorgesehen sein, dass die Zusammensetzung nach der Erfindung (a) das mindestens eine Polysiloxan je Applikations- und/oder Dosiereinheit in einer Menge von 10 mg, insbesondere mindestens 30 mg, vorzugsweise mindestens 50 mg, bevorzugt mindestens 80 mg, besonders bevorzugt mindestens 100 mg, enthält. Gleichermaßen kann es erfindungsgemäß vorgesehen sein, dass die Zusammensetzung nach der Erfindung (a) das mindestens eine Polysiloxan je Applikations- und/oder Dosiereinheit in einer Menge von höchstens 1.000 mg, insbesondere höchstens 800 mg, vorzugsweise höchstens 600 mg, bevorzugt höchstens 400 mg, besonders bevorzugt höchstens 350 mg, enthält.

Wie zuvor angeführt, hat die Anmelderin überraschenderweise herausgefunden, dass die Wirkung (a) des mindestens einen Polysiloxans durch die gezielte Kombination mit sehr speziellen ätherischen Ölen, welche nämlich in Form von (b) Pfefferminzöl, (c) Anisöl, (d) Fenchelöl und (e) Kümmelöl eingesetzt werden, signifikant gesteigert werden kann. In diesem Zusammenhang zeigt gerade die erfindungsgemäß eingesetzte spezielle Kombination von (b) Pfefferminzöl, (c) Anisöl, (d) Fenchelöl und (e) Kümmelöl eine besonders hervorragende Wirksamkeit und Wirkverstärkung in Bezug auf (a) das erfindungsgemäße Polysiloxan.

Bei den erfindungsgemäß eingesetzten ätherischen Ölen in Form von (b) Pfefferminzöl, (c) Anisöl, (d) Fenchelöl und (e) Kümmelöl handelt es sich insbesondere um pflanzlich basierte Karminativa, welche die Prokinetik fördern bzw. welche prokinetisch wirken und somit die Abläufe im Magen/Darm-Trakt positiv beeinflussen, wobei insbesondere spasmolytische Kontraktionen im Magen/Darm-Trakt verringert bzw. verhindert werden, so dass die in Rede stehenden ätherischen Öle eine spasmolytische Wirkung entfalten bzw. aufweisen.

Was das im Rahmen der Zusammensetzung eingesetzte (b) Pfefferminzöl anbelangt, so besitzt dieses insbesondere eine spasmolytische Wirkung insbesondere an der glatten Muskulatur des Magen/Darm-Trakts. Pfefferminzöl weist eine karminative Wirkung auf. Was (b) das Pfefferminzöl weiterhin anbelangt, so kann dieses insbesondere aus den oberirdischen Teilen der Pfefferminze, botanisch *Mentha piperita,* insbesondere aus den Blättern, gewonnen werden. Erfindungsgemäß eingesetztes Pfefferminzöl kann insbesondere Menthol, vorzugsweise in Mengen von 25 bis 45 Gew.-%, Menthon, vorzugsweise in Mengen von 20 bis 30 Gew.-%, und/oder Menthylacetat, vorzugsweise in Mengen von 2 bis 10 Gew.-%, aufweisen, jeweils bezogen auf das ätherische Öl. Für weitergehende Einzelheiten zu dem erfindungsgemäß eingesetzten (b) Pfefferminzöl kann verwiesen werden auf RÖMPP Lexikon Naturstoffe, erste Auflage, Georg Thieme Verlag, Stuttgart/New York, 1997, Seiten 478 und 479, Stichwort: "Pfefferminzöl" und die dort referierte Literatur, deren gesamter Inhalt hiermit durch Bezugnahme eingeschlossen ist.

Im Allgemeinen enthält die erfindungsgemäße Zusammensetzung (b) das Pfefferminzöl in einer Menge von 0,001 bis 1 Gew.-%, insbesondere 0,005 bis 0,5 Gew.-%, vorzugsweise 0,01 bis 0,1 Gew.-%, bevorzugt 0,015 bis 0,08 Gew.-%, besonders bevorzugt 0,02 bis 0,06 Gew.-%, bezogen auf die Zusammensetzung.

In Bezug auf die Zusammensetzung nach der Erfindung kommen auch der entsprechenden Obergrenze und Untergrenze der eingesetzten Menge an (b) Pfefferminzöl eine wichtige Bedeutung zu. So **ist** es erfindungsgemäß vorgesehen, dass die Zusammensetzung nach der Erfindung (b) das Pfefferminzöl in einer Menge von mindestens 0,001 Gew.-%, insbesondere mindestens 0,005 Gew.-%, vorzugsweise mindestens 0,01 Gew.-%, bevorzugt mindestens 0,015 Gew.-%, besonders bevorzugt mindestens 0,02 Gew.-%, bezogen auf die Zusammensetzung, enthält. Gleichermaßen ist es erfindungsgemäß vorgesehen, dass die Zusammensetzung nach der Erfindung (b) das Pfefferminzöl in einer Menge von höchstens 1 Gew.-%, insbesondere höchstens 0,5 Gew.-%, vorzugsweise höchstens 0,1 Gew.-%, bevorzugt höchstens 0,08 Gew.-%, besonders bevorzugt höchstens 0,06 Gew.-%, bezogen auf die Zusammensetzung, enthält.

Darüber hinaus kann es erfindungsgemäß vorgesehen sein, dass die Zusammensetzung nach der Erfindung (b) das Pfefferminzöl je Applikations- und/oder Dosiereinheit in einer Menge von 0,001 bis 1 mg, insbesondere 0,01 bis 0,8 mg, vorzugsweise 0,02 bis 0,6 mg, bevorzugt 0,05 bis 0,5 mg, besonders bevorzugt 0,06 bis 0,25 mg, enthält.

Was weiterhin das im Rahmen der Zusammensetzung nach der Erfindung eingesetzte (c) Anisöl anbelangt, so weist diese Komponente insbesondere auch eine krampflösende und verdauungsfördernde Wirkung auf. Das erfindungsgemäß eingesetzte (c) Anisöl kann dem Fachmann in an sich bekannter Weise insbesondere aus Früchten der Anispflanze, botanisch *Pimpinella anisum,* gewonnen werden. Hauptbestandteil des Anisöls ist trans-Anethol, welches insbesondere in Mengen von 80 bis 90 Gew.-%, bezogen auf das Anisöl, vorliegen kann. Weitere Bestandteile von Anisöl können Cumarine, Estagol, Foeniculin, Vanillin, Anisaldehyd und/oder Anissäure sein.

Erfindungsgemäß enthält die Zusammensetzung nach der Erfindung (c) das Anisöl in Form von Sternanisöl.

Denn diesbezüglich ergeben sich besonders positive Wirkeigenschaften, insbesondere im Hinblick auf die spezielle Kombination mit den weiteren ätherischen Ölen (b) sowie (d) und (e) bzw. dem erfindungsgemäß eingesetzten (a) Polysiloxan.

Sternanisöl kann im Allgemeinen aus dem Echten Sternanis, botanisch *Illicium verum,* gewonnen werden, wobei diesbezüglich insbesondere eine Extraktion aus den reifen Früchten der Pflanze vorgenommen werden kann. Sternanisöl enthält als Hauptkomponente trans-Anethol, insbesondere in einer Menge von 80 bis 90 Gew.-%, bezogen auf das ätherische Öl. Zudem kann Sternanisöl Estragol, Foeniculin, Limonen und/oder Cineol enthalten. Für weitergehende Einzelheiten zu Sternanisöl kann verwiesen werden auf RÖMPP Lexikon Naturstoffe, erste Auflage, Georg Thieme Verlag, Stuttgart/New York, 1997, Seite 609, Stichwort: "Sternanis" und die dort referierte Literatur, deren gesamter Inhalt hiermit durch Bezugnahme eingeschlossen ist.

Was die Menge an (c) Anisöl anbelangt, so kann diese gleichermaßen in weiten Bereichen variieren. Erfindungsgemäß ist es vorgesehen, dass die Zusammensetzung nach der Erfindung (c) das Anisöl in einer Menge von 0,0015 bis 1,5 Gew.-%, insbesondere 0,006 bis 1 Gew.-%, vorzugsweise 0,015 bis 0,15 Gew.-%, bevorzugt 0,02 bis 0,1 Gew.-%, besonders bevorzugt 0,025 bis 0,08 Gew.-%, bezogen auf die Zusammensetzung, enthält.

In diesem Zusammenhang ist es erfindungsgemäß gleichermaßen vorgesehen, dass die Zusammensetzung nach der Erfindung (c) das Anisöl in einer Menge von mindestens 0,0015 Gew.-%, insbesondere mindestens 0,006 Gew.-%, vorzugsweise mindestens 0,015 Gew.-%, bevorzugt mindestens 0,02 Gew.-%, besonders bevorzugt mindestens 0,025 Gew.-%, bezogen auf die Zusammensetzung, enthält. Zudem ist es erfindungsgemäß gleichermaßen auch vorgesehen, dass die Zusammensetzung nach der Erfindung (c) das Anisöl in einer Menge von höchstens 1,5 Gew.-%, insbesondere höchstens 1 Gew.-%, vorzugsweise höchstens 0,15 Gew.-%, bevorzugt höchstens 0,1 Gew.-%, besonders bevorzugt höchstens 0,08 Gew.-%, bezogen auf die Zusammensetzung, enthält.

Im Allgemeinen enthält die Zusammensetzung nach der Erfindung (c) das Anisöl je Applikations- und/oder Dosiereinheit in einer Menge von 0,002 bis 1,5 mg, insbesondere 0,02 bis 1,2 mg, vorzugsweise 0,04 bis 1 mg, bevorzugt 0,05 bis 0,8 mg, besonders bevorzugt 0,06 bis 0,3 mg.

Was weiterhin die im Rahmen der Zusammensetzung nach der Erfindung eingesetzte Komponente (d) auf Basis des Fenchelöls anbelangt, so weist auch dieses eine karminative bzw. spasmolytische Wirkung, insbesondere an der glatten Muskulatur des Magen/Darm-Trakts, auf. Fenchelöl kann insbesondere durch Extraktion von Früchten bzw. Wurzeln beispielsweise aus dem Gemeinen Fenchel, botanisch *Foeniculum vulgare,* gewonnen werden. Fenchelöl weist als Hauptbestandteil Anethol auf, insbesondere in einer Menge von mehr als 80 Gew.-%, bezogen auf das ätherische Öl. Darüber hinaus kann Fenchelöl auch Estragol und/oder Fenchon aufweisen, insbesondere in einer Menge von 1 bis 2 Gew.-%, bezogen auf das ätherische Öl. Für weitergehende Einzelheiten zu Fenchelöl kann verwiesen werden auf RÖMPP Lexikon Naturstoffe, erste Auflage, Georg Thieme Verlag, Stuttgart/New York, 1997, Seite 221, Stichwort: "Fenchelöl", und die dort referierte Literatur, deren gesamter Inhalt hiermit durch Bezugnahme eingeschlossen ist.

Im Rahmen der vorliegenden Erfindung ist es vorgesehen, dass die Zusammensetzung nach der Erfindung (d) das Fenchelöl in Form von Bitterfenchelöl enthält, welches insbesondere aus Bitterfenchel, botanisch *Foeniculum vulgare* ssp. *piperitum,* gewonnen werden kann.

In diesem Zusammenhang ergeben sich besonders positive Effekte hinsichtlich der Wirksamkeit der erfindungsgemäßen Zusammensetzung im Hinblick auf die zugrundeliegenden Indikationen bzw. Symptome, da die Zusammensetzung nach der Erfindung (c) das Anisöl in Form von Sternanisöl und (d) das Fenchelöl in Form von Bitterfenchelöl enthält.

Bitterfenchelöl unterscheidet sich in seiner Zusammensetzung von Fenchelöl insbesondere insofern, als Bitterfenchelöl als Hauptbestandteil Anethol insbesondere in einer Menge von 30 bis 40 Gew.-% aufweist. Zudem weist Bitterfenchelöl alpha-Phellandren und/oder Fenchon, insbesondere in einer Menge von jeweils 5 bis 20 Gew.-%, und Limonen, insbesondere in einer Menge von etwa 20 Gew.-%, auf, jeweils bezogen auf das ätherische Öl.

Die Menge an (d) Fenchelöl in der erfindungsgemäßen Zusammensetzung kann in weiten Bereichen variieren. In diesem Zusammenhang ist es im Rahmen der vorliegenden Erfindung vorgesehen, dass die Zusammensetzung nach der Erfindung (d) das Fenchelöl in einer Menge von 0,002 bis 1,3 Gew.-%, insbesondere 0,006 bis 0,7 Gew.-%, vorzugsweise 0,015 bis 0,12 Gew.-%, bevorzugt 0,02 bis 0,1 Gew.-%, besonders bevorzugt 0,025 bis 0,08 Gew.-%, bezogen auf die Zusammensetzung, enthält.

Auch in Bezug auf (d) das Fenchelöl spielen die jeweiligen Unter- und Obergrenzen bezüglich der eingesetzten Mengen eine wichtige Rolle: So ist es im Rahmen der vorliegenden Erfindung vorgesehen, dass die Zusammensetzung nach der Erfindung (d) das Fenchelöl in einer Menge von mindestens 0,002 Gew.-%, insbesondere mindestens 0,006 Gew.-%, vorzugsweise mindestens 0,015 Gew.-%, bevorzugt mindestens 0,02 Gew.-%, besonders bevorzugt 0,025 Gew.-%, bezogen auf die Zusammensetzung, enthält. Zudem ist es erfindungsgemäß vorgesehen, dass die Zusammensetzung nach der Erfindung (d) das Fenchelöl in einer Menge von höchstens 1,3 Gew.-%, insbesondere höchstens 0,7 Gew.-%, vorzugsweise höchstens 0,12 Gew.-%, bevorzugt höchstens 0,1 Gew.-%, besonders bevorzugt höchstens 0,08 Gew.-%, bezogen auf die Zusammensetzung, enthält.

Darüber hinaus kann die Zusammensetzung nach der Erfindung (d) das Fenchelöl je Applikations- und/oder Dosiereinheit in einer Menge von 0,0015 bis 1,3 mg, insbesondere 0,015 bis 1 mg, vorzugsweise 0,03 bis 0,8 mg, bevorzugt 0,05 bis 0,7 mg, besonders bevorzugt 0,06 bis 0,25 mg, enthalten.

Was zudem das erfindungsgemäß eingesetzte (e) Kümmelöl anbelangt, welches gleichermaßen eine karminative Wirkung aufweist, so kann diese Komponente beispielsweise durch Extraktion, insbesondere der Samen, von Kümmel, botanisch *Carum carvi,* gewonnen werden. Kümmelöl ist ein farbloses bis gelbliches ätherisches Öl, welches als Hauptinhaltsstoffe insbesondere Carvon, insbesondere in Mengen von 50 bis 60 Gew.-%, bezogen auf das ätherische Öl, und/oder Limonen, insbesondere in Mengen von 30 bis 45 Gew.-%, bezogen auf das ätherische Öl, aufweist. Weitere Inhaltsstoffe von Kümmelöl sind Myrcen, alpha-Phellandren, beta-Cymol, beta-Carophyllen, cis- und/oder trans-Carveol, cis- und/oder trans-Dihydrocarvon, trans-Dihydrocarveol sowie alpha- und/oder beta-Pinen. Die vorgenannten Inhaltsstoffe begründen die karminative Wirkung von Kümmelöl bei dyspeptischen Beschwerden, insbesondere bei Völlegefühl und Blähungen. Neben der spasmolytischen Wirkung an der glatten Muskulatur des Magen/Darm-Trakts besitzt Kümmelöl darüber hinaus auch antimikrobielle Eigenschaften. Für weitergehende Einzelheiten zu Kümmel bzw. Kümmelöl kann verwiesen werden auf RÖMPP Chemielexikon, 10. Auflage, Georg Thieme Verlag, Stuttgart/New York, Band 3, 1997, Seite 2295, Stichwort: "Kümmel" und Stichwort: "Kümmelöl", sowie die dort jeweils referierte Literatur, deren gesamter Inhalt hiermit durch Bezugnahme eingeschlossen ist.

Was die Menge an (e) Kümmelöl in der erfindungsgemäßen Zusammensetzung anbelangt, so kann diese gleichermaßen in weiten Bereichen variieren. Erfindungsgemäß enthält die Zusammensetzung nach der Erfindung (e) das Kümmelöl in einer Menge von 0,0005 bis 0,8 Gew.-%, insbesondere 0,001 bis 0,4 Gew.-%, vorzugsweise 0,005 bis 0,1 Gew.-%, bevorzugt 0,01 bis 0,06 Gew.-%, besonders bevorzugt 0,015 bis 0,05 Gew.-%, bezogen auf die Zusammensetzung.

Erfindungsgemäß enthält die Zusammensetzung nach der Erfindung (e) das Kümmelöl in einer Menge von mindestens 0,0005 Gew.-%, insbesondere mindestens 0,001 Gew.-%, vorzugsweise mindestens 0,005 Gew.-%, bevorzugt mindestens 0,01 Gew.-%, besonders bevorzugt mindestens 0,015 Gew.-%, bezogen auf die Zusammensetzung. Gleichermaßen ist es erfindungsgemäß vorgesehen, dass die Zusammensetzung nach der Erfindung (e) das Kümmelöl in einer Menge von höchstens 0,8 Gew.-%, insbesondere höchstens 0,4 Gew.-%, vorzugsweise höchstens 0,1 Gew.-%, bevorzugt höchstens 0,06 Gew.-%, besonders bevorzugt höchstens 0,05 Gew.-%, bezogen auf die Zusammensetzung, enthält.

Darüber hinaus kann es erfindungsgemäß vorgesehen sein, dass die Zusammensetzung nach der Erfindung (e) das Kümmelöl je Applikations- und/oder Dosiereinheit in einer Menge von 0,001 bis 0,8 mg, insbesondere 0,005 bis 0,6 mg, vorzugsweise 0,01 bis 0,5 mg, bevorzugt 0,02 bis 0,2 mg, besonders bevorzugt 0,03 bis 0,1 mg, enthält.

Wie zuvor angeführt, führt die sehr spezielle Kombination von ätherischen Ölen auf Basis der Komponenten (b) bis (e) in völlig überraschender Weise zu einer hervorragenden Wirksamkeit der erfindungsgemäßen Zusammensetzung in Bezug auf die Behandlung von Verdauungsstörungen und einer effektiven Linderung der damit einhergehenden Symptome. Insbesondere führt die gezielte Kombination der erfindungsgemäß eingesetzten speziellen ätherischen Öle auf Basis von (b) Pfefferminzöl, (c) Anisöl, (d) Fenchelöl und (e) Kümmelöl zu einer überraschenden Wirkverstärkung, insbesondere im Hinblick auf die weiterführende Kombination mit dem erfindungsgemäß eingesetzten (a) Polysiloxan, so dass insgesamt eine Zusammensetzung nach der Erfindung bereitgestellt wird, welche über hervorragende karminative Wirkungen verfügt und dabei gleichermaßen gut verträglich ist. Dabei wird die Wirksamkeit der erfindungsgemäßen Zusammensetzung, wie zuvor angeführt, durch die spezielle Verwendung von (c) Anisöl in Form von Sternanisöl und/oder von (d) Fenchelöl in Form von Bitterfenchelöl nochmals gesteigert. Darüber hinaus sind die vorgenannten pflanzlichen bzw. ätherischen Öle auf Basis der Komponenten (b) bis (e) in Bezug auf die Geschmacks- bzw. Geruchseigenschaften der erfindungsgemäßen pharmazeutischen Zusammensetzung förderlich.

Auch das Verhältnis der für die Zusammensetzung nach der Erfindung eingesetzten Komponenten bzw. Wirksubstanzen untereinander spielt hinsichtlich der Wirksamkeit der erfindungsgemäßen Zusammensetzung eine Rolle: So kann die erfindungsgemäße Zusammensetzung ein Verhältnis (a) Polysiloxan einerseits zu (b) Pfefferminzöl und (c) Anisöl und (d) Fenchelöl und (e) Kümmelöl andererseits [(a) : ((b) + (c) + (d) + (e)] im Bereich von [(10 bis 1.000) : (0,1 bis 5)], insbesondere [(100 bis 300): (0,2 bis 2)], aufweisen.

Weiterhin kann die Zusammensetzung nach der Erfindung ein Verhältnis (a) Polysiloxan zu (b) Pfefferminzöl zu (c) Anisöl zu (d) Fenchelöl zu (e) Kümmelöl [(a) : (b) : (c) : (d) : (e)] im Bereich von [(10 bis 1.000): (0,01 bis 1): (0,015 bis 1,5) : (0,013 bis 1,3): (0,005 bis 0,8)], insbesondere [(100 bis 300):(0,05 bis 0,5) : (0,06 bis 0,6) : (0,055 bis 0,55) : (0,02 bis 0,3)], aufweisen.

Auch dem Verhältnis der ätherischen Öle auf Basis der Komponenten (b) bis (e) untereinander kommt hinsichtlich der Wirksamkeit der erfindungsgemäßen Zusammensetzung eine Bedeutung zu: So kann es erfindungsgemäß vorgesehen sein, dass die Zusammensetzung nach der Erfindung ein Verhältnis (b) Pfefferminzöl zu (c) Anisöl zu (d) Fenchelöl zu (e) Kümmelöl [(b) : (c) : (d) : (e)] im Bereich von [(0,01 bis 1) : (0,015 bis 1,5): (0,013 bis 1,3) : (0,005 bis 0,8)], insbesondere [(0,05 bis 0,5) : (0,06 bis 0,6) : (0,055 bis 0,55) : (0,02 bis 0,3)], aufweist.

Gemäß einer erfindungsgemäß bevorzugten Ausführungsform besteht die Zusammensetzung nach der Erfindung aus (a) dem mindestens einen Polysiloxan, (b) Pfefferminzöl, (c) Anisöl, (d) Fenchelöl und (e) Kümmelöl. Mit anderen Worten ist die erfindungsgemäße Zusammensetzung gemäß dieser erfindungsgemäß bevorzugten Ausführungsform frei von weiteren Zusatz- bzw. Hilfsstoffen und/oder weiteren Wirkkomponenten. Diesbezüglich ist es erfindungsgemäß vorgesehen, dass die Zusammensetzung nach der Erfindung frei von Enzymen ist bzw. dass die Zusammensetzung nach der Erfindung enzymfrei ausgebildet ist.

Sofern dies anwendungsbezogen bzw. konfektionstechnisch erwünscht bzw. erforderlich ist, kann es - auch wenn dies erfindungsgemäß weniger bevorzugt ist - jedoch auch vorgesehen sein, dass die Zusammensetzung nach der Erfindung außerdem mindestens einen pharmazeutischen Träger enthält.

In diesem Zusammenhang kann es zudem vorgesehen sein, dass die erfindungsgemäße Zusammensetzung außerdem noch weitere Inhaltsstoffe enthält, beispielsweise Zusatz- und/oder Hilfsstoffe. Solche Zusatz- und/oder Hilfsstoffe können beispielsweise ausgewählt sein aus der Gruppe von Füll-, Streck-, Binde-, Netz-, Stabilisierungs-, Färbe-, Puffer-, Riech-, Geschmacks-, Süßungs-, Aroma-, Verarbeitungshilfs- und/oder Konservierungsstoffen bzw. -mitteln sein. Wie zuvor angeführt, kann sich diese erfindungsgemäß weniger bevorzugte Ausführungsform vorrangig aus anwendungsbezogenen bzw. produktionstechnischen Erwägungen ergeben, insbesondere sofern spezielle Produkteigenschaften, wie beispielsweise Darreichungsform, Haltbarkeit und/oder organoleptische Eigenschaften, gezielt eingestellt werden sollen.

Die für die erfindungsgemäße Zusammensetzung eingesetzten Komponenten (a) bis (e) angeführten Mengenangaben beziehen sich, wie zuvor angeführt, jedoch auch in diesem Fall auf die Zusammensetzung nach der Erfindung als solche auf Basis der Summe der in Rede stehenden Komponenten (a) bis (e), d. h. ohne weitere Zusatzstoffe als solche, welche dann gewissermaßen der Zusammensetzung nach der Erfindung auf Basis der Komponenten (a) bis (e) zugegeben sind. Erfindungsgemäß kann die Zusammensetzung in peroral verabreichbarer Form vorliegen.

Weiterhin kann die Zusammensetzung nach der Erfindung flüssig und/oder viskos sein. Insbesondere kann die Zusammensetzung nach der Erfindung als Dispersion, insbesondere Suspension und/oder Emulsion, vorzugsweise Emulsion, vorliegen.

Die Zusammensetzung nach der Erfindung kann insbesondere als flüssig basierte Dosiereinheit vorliegen. In diesem Zusammenhang kann die Zusammensetzung nach der Erfindung insbesondere als Dosiereinheit auf Basis einer Suspension, bevorzugt in Form von Dosiereinheiten für die einmalige Applikation, vorliegen. Bei den in Rede stehenden Dosiereinheiten kann es sich insbesondere um Kapseln mit flüssigem bzw. viskosem Inhalt auf Basis der erfindungsgemäßen Zusammensetzung, vorzugsweise um Weichkapseln oder aber um Portionsbeutel mit flüssigem bzw. viskosem Inhalt auf Basis der erfindungsgemäßen Zusammensetzung oder dergleichen handeln, welche die erfindungsgemäße Zusammensetzung aufweisen.

In diesem Zusammenhang kann die Zusammensetzung insbesondere in Dosiereinheiten auf Basis von jeweils 0,05 bis 5 g, insbesondere 0,1 bis 4 g, vorzugsweise 0,2 bis 2 g der erfindungsgemäßen Zusammensetzung vorliegen.

Insbesondere kann die Zusammensetzung nach der Erfindung in ein Hüllmaterial und/oder Kapselmaterial, insbesondere Weichkapselmaterial eingebracht vorliegen.

Insbesondere kann das Hüllmaterial bzw. Kapselmaterial magensaftlöslich ausgebildet sein. Gleichermaßen kann es erfindungsgemäß auch vorgesehen sein, dass das Kapselmaterial, in welchem die Zusammensetzung nach der Erfindung eingebracht vorliegt, darmlöslich, vorzugsweise dünndarmlöslich ausgebildet ist. Die vorgenannten Ausführungsformen sind insbesondere mit dem Vorteil verbunden, dass die Wirkstoffe erst unmittelbar am gewünschten Wirkort freigesetzt werden.

In diesem Zusammenhang kann das Hüllmaterial bzw. Kapselmaterial auf Basis von Gelatine ausgebildet sein. Zudem kann das Hüllmaterial bzw. Kapselmaterial insbesondere modifizierte Maisstärke, mindestens einen mehrwertigen Alkohol, insbesondere Glycerol, und/oder mindestens ein Polysaccharid, insbesondere Carrageenan, aufweisen.

Insbesondere kann die erfindungsgemäße Zusammensetzung demnach insbesondere in einer Kapsel, insbesondere einer Weichkapsel, vorzugsweise auf Basis von Gelatine, eingebracht vorliegen. Dies ist mit dem Vorteil verbunden, dass die erfindungsgemäße Zusammensetzung in einfacher Weise und dosierfertig appliziert werden kann, wobei zudem die organoleptischen Eigenschaften verbessert sind.

Die erfindungsgemäße Zusammensetzung kann im Allgemeinen als Arzneimittel oder Pharmazeutikum, als Medizinprodukt, als Homöopathikum oder als Nahrungsergänzungsmittel, Nahrungsmittelzusatz oder Diätetikum ausgebildet sein.

Gemäß einer weiteren Ausführungsform der vorliegenden Erfindung betrifft die vorliegende Erfindung eine Zusammensetzung, insbesondere pharmazeutische Zubereitung, vorzugsweise zur therapeutischen oder prophylaktischen Behandlung von Verdauungsbeschwerden oder -störungen, insbesondere Verdauungsfehlfunktionen, Meteorismus, Flatulenz, Koliken, Völlegefühl, Magendruck, übermäßiger Gasbildung im Magen/Darm-Trakt oder dergleichen, insbesondere wie zuvor definiert, wobei die Zusammensetzung - in Kombination und jeweils in wirksamen Mengen, insbesondere pharmazeutisch wirksamen Mengen -
(a) mindestens ein Polysiloxan in einer Menge von 60 bis 99,9 Gew.-%, besonders bevorzugt 70 bis 99,85 Gew.-%;
(b) Pfefferminzöl in einer Menge von 0,001 bis 1 Gew.-%, insbesondere 0,005 bis 0,5 Gew.-%, vorzugsweise 0,01 bis 0,1 Gew.-%, bevorzugt 0,015 bis 0,08 Gew.-%, besonders bevorzugt 0,02 bis 0,06 Gew.-%;
(c) Anisöl in Form von Sternanisöl in einer Menge von 0,0015 bis 1,5 Gew.-%, insbesondere 0,006 bis 1 Gew.-%, vorzugsweise 0,015 bis 0,15 Gew.-%, bevorzugt 0,02 bis 0,1 Gew.-%, besonders bevorzugt 0,025 bis 0,08 Gew.-%;
(d) Fenchelöl in Form von Bitterfenchelöl in einer Menge von 0,002 bis 1,3 Gew.-%, insbesondere 0,006 bis 0,7 Gew.-%, vorzugsweise 0,015 bis 0,12 Gew.-%, bevorzugt 0,02 bis 0,1 Gew.-%, besonders bevorzugt 0,025 bis 0,08 Gew.-%;
(e) Kümmelöl in einer Menge von 0,0005 bis 0,8 Gew.-%, insbesondere 0,001 bis 0,4 Gew.-%, vorzugsweise 0,005 bis 0,1 Gew.-%, bevorzugt 0,01 bis 0,06 Gew.-%, besonders bevorzugt 0,015 bis 0,05 Gew.-%,
jeweils bezogen auf die Zusammensetzung, enthält,
wobei die Zusammensetzung ein Verhältnis (a) Polysiloxan zu (b) Pfefferminzöl zu (c) Anisöl zu (d) Fenchelöl zu (e) Kümmelöl [(a) : (b) : (c) : (d) : (e)] im Bereich von [(10 bis 1.000): (0,01 bis 1): (0,015 bis 1,5): (0,013 bis 1,3) : (0,005 bis 0,8)], insbesondere [(100 bis 300) : (0,05 bis 0,5) : (0,06 bis 0,6) : (0,055 bis 0,55): (0,02 bis 0,3)], aufweist und wobei die Zusammensetzung enzymfrei ausgebildet ist.

Die Herstellung der erfindungsgemäßen Zusammensetzung bzw. der zugrundeliegenden ätherischen Öle liegt im fachüblichen Können des Fachmanns. Für weitere diesbezügliche Ausführungen kann zudem auf das Ausführungsbeispiel verwiesen werden.

Im Rahmen der vorliegenden Erfindung ist es im Ergebnis gelungen, eine Zusammensetzung auf Basis des gezielten Einsatzes mindestens eines Polysiloxans und einer sehr speziellen Kombination von ätherischen Ölen bereitzustellen, welche in Bezug auf die vorgenannten Verdauungsfehlfunktionen bzw. -störungen bei gleichzeitig guter Verträglichkeit wirksam ist. Die erfindungsgemäße Zusammensetzung, insbesondere pharmazeutische Zubereitung, kombiniert in sinnvoller Weise eine Polysiloxankomponente mit einer speziellen Kombination sehr spezieller ätherischer Öle, wodurch die karminative Wirkung der erfindungsgemäßen Zusammensetzung gezielt unterstützt und gefördert wird und so den zuvor genannten Verdauungsbeschwerden bzw. -störungen wirksam entgegengewirkt wird. Im Rahmen der erfindungsgemäß eingesetzten Kombination der zugrundeliegenden ätherischen Öle mit dem Polysiloxan wird dabei eine über die Wirkung der jeweiligen Einzelstoffe hinausgehende Wirkung erzielt, was auf einen gewissen synergistischen Effekt zurückgeführt werden kann. In diesem Zusammenhang ergänzen sich insbesondere die spasmolytischen bzw. karminativen Eigenschaften der eingesetzten ätherischen Öle und die oberflächenspannungsmodifizierenden Eigenschaften des eingesetzten Polysiloxans in signifikanter Weise.

Weiterhin betrifft die vorliegende Erfindung - gemäß einem zweiten Aspekt der vorliegenden Erfindung - eine Dosiereinheit, insbesondere zur peroralen Applikation, insbesondere in Form einer Kapsel, insbesondere Weichkapsel, welche die Zusammensetzung nach der Erfindung, wie zuvor definiert, enthält. Im Hinblick auf die diesbezüglich einsetzbaren Kapselmaterialien bzw. die Ausbildung der Kapsel als solche kann auf die im Zusammenhang mit der erfindungsgemäßen Zusammensetzung angeführten Ausführungen verwiesen werden.

Zudem betrifft die vorliegende Erfindung auch eine Dosiereinheit, insbesondere zur peroralen Applikation, insbesondere in Form einer vorzugsweise dosierfertigen Umverpackung, insbesondere Portionsbeutel, für die einmalige Applikation, welche die erfindungsgemäße Zusammensetzung, wie zuvor definiert, enthält.

Die vorliegende Erfindung betrifft zudem - gemäß einem dritten Aspekt der vorliegenden Erfindung - eine Verpackungseinheit, welche die erfindungsgemäße Dosiereinheit bzw. eine Vielzahl der Dosiereinheiten nach der Erfindung, wie zuvor definiert, enthält.

Darüber hinaus eignet sich die erfindungsgemäße Zusammensetzung für die Verwendung zur therapeutischen oder prophylaktischen Behandlung von Verdauungsbeschwerden oder -störungen, insbesondere Verdauungsfehlfunktionen, Meteorismus, Flatulenz, Koliken, Völlegefühl, Magendruck, übermäßige Gasbildung im Magen/Darm-Trakt oder dergleichen.

In diesem Zusammenhang kann die Zusammensetzung bzw. Kombination nach der Erfindung beispielsweise etwa 10 bis 60 min, vorzugsweise etwa 30 min, vor einer Mahlzeit oder mit der Mahlzeit peroral appliziert werden (z. B. mit etwas Flüssigkeit unzerkaut geschluckt werden, wenn es sich um Kapseln, insbesondere Weichkapseln, handelt). Dabei kann die Zusammensetzung nach der Erfindung derart hergerichtet werden, dass pro Applikation bzw. Verabreichung 0,1 bis 10 g, insbesondere 0,25 bis 5 g, vorzugsweise 0,5 bis 2,5 g, der erfindungsgemäßen Zusammensetzung appliziert werden.

In diesem Zusammenhang kann die erfindungsgemäße Zusammensetzung als Karminativum und/oder zur Herstellung eines Arzneimittels mit karminativer Wirkung verwendet werden.

Schließlich betrifft die vorliegende Erfindung - gemäß einem vierten Aspekt der vorliegenden Erfindung - ein Karminativum, welches die erfindungsgemäße Zusammensetzung, wie zuvor definiert, und/oder die erfindungsgemäße Dosiereinheit, wie zuvor definiert, und/oder die erfindungsgemäße Verpackungseinheit, wie zuvor definiert, umfasst.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt. Das nachfolgende Ausführungsbeispiel dient lediglich der Veranschaulichung der vorliegenden Erfindung, ohne sich jedoch hierauf zu beschränken.

### Ausführungsbeispiel:

### 1. Erfindungsgemäße Zusammensetzung:

Eine Weichkapsel auf Basis der erfindungsgemäßen Zusammensetzung wird unter Einsatz der nachfolgenden Komponenten bzw. Inhaltsstoffe hergestellt:

| Inhaltsstoffe: | Menge in mg pro Tablette: |
|---|---|
| Erfindungsgemäße Zusammensetzung: | |
| Simeticon | 250,57 |
| Pfefferminzöl | 0,10 |
| Sternanisöl | 0,15 |
| Bitterfenchelöl | 0,13 |
| Kümmelöl | 0,05 |
| Füllgewicht: | 251,00 |
| | |

| Kapselmaterial: | Menge in mg pro Kapselhülle: |
|---|---|
| modifizierte Maisstärke | 54,6 |
| Glycerol | 42,5 |
| Carrageenan | 17,7 |
| Natriumhydrogenphosphat | 1,6 |
| gereinigtes Wasser | 6,6 |

Die Herstellung der erfindungsgemäßen Zusammensetzung erfolgt in dem Fachmann an sich bekannter Weise, wobei die zugrundeliegenden Komponenten insbesondere zusammengeführt und miteinander vermischt werden. Die so resultierende Flüssigkeit wird gleichermaßen in an sich bekannter Weise in das auf übliche Weise hergestellte Kapselmaterial eingebracht.

### 2. Anwendungs- und Wirksamkeitsstudien:

Erfindungsgemäße Kapseln, welche je Kapsel 250,57 mg Simeticon, 0,1 mg Pfefferminzöl, 0,15 mg Sternanisöl, 0,13 mg Bitterfenchelöl und 0,05 mg Kümmelöl enthielten, wurden an 15 Patienten mit Verdauungsbeschwerden etwa 30 min vor jeder Mahlzeit verabreicht (ein bis zwei Tabletten pro Mahlzeit mit etwas Flüssigkeit eingenommen). Unter Verabreichung der erfindungsgemäßen Kapseln waren die Patienten auch nach fettreichen Mahlzeiten nahezu vollständig frei von Verdauungsproblemen, wobei insbesondere die mit den Verdauungsproblemen einhergehenden Symptome, wie Völlegefühl, Blähungen und Magendruck, deutlich minimiert wurden.

Die vorgenannte Anwendungs- bzw. Wirksamkeitsstudie zeigt somit die hervorragende verdauungsfördernde Wirkung der erfindungsgemäßen Zusammensetzung, insbesondere was die effektive Verminderung von Verdauungsfehlfunktionen und den damit einhergehenden Symptomen anbelangt.

## Patentansprüche

1. Zusammensetzung, insbesondere pharmazeutische Zubereitung, vorzugsweise zur Verwendung zur therapeutischen oder prophylaktischen Behandlung von Verdauungsbeschwerden oder -störungen, insbesondere Verdauungsfehlfunktionen, Meteorismus, Flatulenz, Koliken, Völlegefühl, Magendruck, übermäßiger Gasbildung im Magen/Darm-Trakt oder dergleichen,
wobei die Zusammensetzung - in Kombination und jeweils in wirksamen Mengen, insbesondere pharmazeutisch wirksamen Mengen -
(a) mindestens ein Polysiloxan in einer Menge von 60 bis 99,9 Gew.-%, bezogen auf die Zusammensetzung;
(b) Pfefferminzöl in einer Menge von 0,001 bis 1 Gew.-%, bezogen auf die Zusammensetzung;
(c) Anisöl in Form von Sternanisöl
und in einer Menge von 0,0015 bis 1,5 Gew.-%, bezogen auf die Zusammensetzung;
(d) Fenchelöl in Form von Bitterfenchelöl
und in einer Menge von 0,002 bis 1,3 Gew.-%, bezogen auf die Zusammensetzung;
(e) Kümmelöl in einer Menge von 0,0005 bis 0,8 Gew.-%, bezogen auf die
Zusammensetzung;
enthält, wobei die Zusammensetzung enzymfrei ausgebildet ist.

2. Zusammensetzung nach Anspruch 1, wobei (a) das Polysiloxan ein Polysiloxan mit oberflächenspannungsmodifizierender und/oder entschäumender Wirkung ist, und/oder wobei (a) das Polysiloxan ein vorzugsweise lineares Alkyl- oder Arylpolysiloxan ist, und/oder wobei (a) das Polysiloxan Simeticon und/oder Dimeticon, vorzugsweise Simeticon, ist.

3. Zusammensetzung nach Anspruch 1 oder 2,
enthaltend (a) das mindestens eine Polysiloxan in einer Menge von 70 bis 99,85 Gew.-%, bezogen auf die Zusammensetzung, und/oder
enthaltend (a) das mindestens eine Polysiloxan je Applikations- und/oder Dosiereinheit in einer Menge von 10 bis 1.000 mg, insbesondere 30 bis 800 mg, vorzugsweise 50 bis 600 mg, bevorzugt 80 bis 400 mg, besonders bevorzugt 100 bis 350 mg, und/oder
enthaltend (a) das mindestens eine Polysiloxan je Applikations- und/oder Dosiereinheit in einer Menge von mindestens 10 mg, insbesondere mindestens 30 mg, vorzugsweise mindestens 50 mg, bevorzugt mindestens 80 mg, besonders bevorzugt mindestens 100 mg, und/oder enthaltend (a) das mindestens eine Polysiloxan je Applikations- und/oder Dosiereinheit in einer Menge von höchstens 1.000 mg, insbesondere höchstens 800 mg, vorzugsweise höchstens 600 mg, bevorzugt höchstens 400 mg, besonders bevorzugt höchstens 350 mg, und/oder
enthaltend (a) das mindestens eine Polysiloxan je Applikations- und/oder Dosiereinheit in einer Menge von mindestens 10 mg, insbesondere mindestens 30 mg, vorzugsweise mindestens 50 mg, bevorzugt mindestens 80 mg, besonders bevorzugt mindestens 100 mg, und/oder enthaltend (a) das mindestens eine Polysiloxan je Applikations- und/oder Dosiereinheit in einer Menge von höchstens 1.000 mg, insbesondere höchstens 800 mg, vorzugsweise höchstens 600 mg, bevorzugt höchstens 400 mg, besonders bevorzugt höchstens 350 mg.

4. Zusammensetzung nach einem der vorangehenden Ansprüche,
enthaltend (b) das Pfefferminzöl in einer Menge von 0,005 bis 0,5 Gew.-%, vorzugsweise 0,01 bis 0,1 Gew.-%, bevorzugt 0,015 bis 0,08 Gew.-%, besonders bevorzugt 0,02 bis 0,06 Gew.-%, bezogen auf die Zusammensetzung, und/oder
enthaltend (b) das Pfefferminzöl in einer Menge von mindestens 0,001 Gew.-%, insbesondere mindestens 0,005 Gew.-%, vorzugsweise mindestens 0,01 Gew.-%, bevorzugt mindestens 0,015 Gew.-%, besonders bevorzugt mindestens 0,02 Gew.-%, bezogen auf die Zusammensetzung, und/oder enthaltend (b) das Pfefferminzöl in einer Menge von höchstens 1 Gew.-%, insbesondere höchstens 0,5 Gew.-%, vorzugsweise höchstens 0,1 Gew.-%, bevorzugt höchstens 0,08 Gew.-%, besonders bevorzugt höchstens 0,06 Gew.-%, bezogen auf die Zusammensetzung, und/oder
enthaltend (b) das Pfefferminzöl je Applikations- und/oder Dosiereinheit in einer Menge von 0,001 bis 1 mg, insbesondere 0,01 bis 0,8 mg, vorzugsweise 0,02 bis 0,6 mg, bevorzugt 0,05 bis 0,5 mg, besonders bevorzugt 0,06 bis 0,25 mg.

5. Zusammensetzung nach einem der vorangehenden Ansprüche,
enthaltend (c) das Anisöl in einer Menge von 0,006 bis 1 Gew.-%, vorzugsweise 0,015 bis 0,15 Gew.-%, bevorzugt 0,02 bis 0,1 Gew.-%, besonders bevorzugt 0,025 bis 0,08 Gew.-%, bezogen auf die Zusammensetzung, und/oder
enthaltend (c) das Anisöl in einer Menge von mindestens 0,0015 Gew.-%, insbesondere mindestens 0,006 Gew.-%, vorzugsweise mindestens 0,015 Gew.-%, bevorzugt mindestens 0,02 Gew.-%, besonders bevorzugt mindestens 0,025 Gew.-%, bezogen auf die Zusammensetzung, und/oder enthaltend (c) das Anisöl in einer Menge von höchstens 1,5 Gew.-%, insbesondere höchstens 1 Gew.-%, vorzugsweise höchstens 0,15 Gew.-%, bevorzugt höchstens 0,1 Gew.-%, besonders bevorzugt höchstens 0,08 Gew.-%, bezogen auf die Zusammensetzung, und/oder
enthaltend (c) das Anisöl je Applikations- und/oder Dosiereinheit in einer Menge von 0,002 bis 1,5 mg, insbesondere 0,02 bis 1,2 mg, vorzugsweise 0,04 bis 1 mg, bevorzugt 0,05 bis 0,8 mg, besonders bevorzugt 0,06 bis 0,3 mg.

6. Zusammensetzung nach einem der vorangehenden Ansprüche,
enthaltend (d) das Fenchelöl in einer Menge von 0,006 bis 0,7 Gew.-%, vorzugsweise 0,015 bis 0,12 Gew.-%, bevorzugt 0,02 bis 0,1 Gew.-%, besonders bevorzugt 0,025 bis 0,08 Gew.-%, bezogen auf die Zusammensetzung, und/oder
enthaltend (d) das Fenchelöl in einer Menge von mindestens 0,002 Gew.-%, insbesondere mindestens 0,006 Gew.-%, vorzugsweise mindestens 0,015 Gew.-%, bevorzugt mindestens 0,02 Gew.-%, besonders bevorzugt 0,025 Gew.-%, bezogen auf die Zusammensetzung, und/oder enthaltend (d) das Fenchelöl in einer Menge von höchstens 1,3 Gew.-%, insbesondere höchstens 0,7 Gew.-%, vorzugsweise höchstens 0,12 Gew.-%, bevorzugt höchstens 0,1 Gew.-%, besonders bevorzugt höchstens 0,08 Gew.-%, bezogen auf die Zusammensetzung, und/oder
enthaltend (d) das Fenchelöl je Applikations- und/oder Dosiereinheit in einer Menge von 0,0015 bis 1,3 mg, insbesondere 0,015 bis 1 mg, vorzugsweise 0,03 bis 0,8 mg, bevorzugt 0,05 bis 0,7 mg, besonders bevorzugt 0,06 bis 0,25 mg.

7. Zusammensetzung nach einem der vorangehenden Ansprüche,
enthaltend (e) das Kümmelöl in einer Menge von 0,001 bis 0,4 Gew.-%, vorzugsweise 0,005 bis 0,1 Gew.-%, bevorzugt 0,01 bis 0,06 Gew.-%, besonders bevorzugt 0,015 bis 0,05 Gew.-%, bezogen auf die Zusammensetzung, und/oder
enthaltend (e) das Kümmelöl in einer Menge von mindestens 0,0005 Gew.-%, insbesondere mindestens 0,001 Gew.-%, vorzugsweise mindestens 0,005 Gew.-%, bevorzugt mindestens 0,01 Gew.-%, besonders bevorzugt mindestens 0,015 Gew.-%, bezogen auf die Zusammensetzung, und/oder enthaltend (e) das Kümmelöl in einer Menge von höchstens 0,8 Gew.-%, insbesondere höchstens 0,4 Gew.-%, vorzugsweise höchstens 0,1 Gew.-%, bevorzugt höchstens 0,06 Gew.-%, besonders bevorzugt höchstens 0,05 Gew.-%, bezogen auf die Zusammensetzung, und/oder
enthaltend (e) das Kümmelöl je Applikations- und/oder Dosiereinheit in einer Menge von 0,001 bis 0,8 mg, insbesondere 0,005 bis 0,6 mg, vorzugsweise 0,01 bis 0,5 mg, bevorzugt 0,02 bis 0,2 mg, besonders bevorzugt 0,03 bis 0,1 mg.

8. Zusammensetzung nach einem der vorangehenden Ansprüche,
wobei die Zusammensetzung ein Verhältnis (a) Polysiloxan einerseits zu (b) Pfefferminzöl und (c) Anisöl und (d) Fenchelöl und (e) Kümmelöl andererseits [(a) : ((b) + (c) + (d) + (e))] im Bereich von [(10 bis 1.000): (0,1 bis 5)], insbesondere [(100 bis 300): (0.2 bis 2)], aufweist, und/oder
wobei die Zusammensetzung ein Verhältnis (a) Polysiloxan zu (b) Pfefferminzöl zu (c) Anisöl zu (d) Fenchelöl zu (e) Kümmelöl [(a) : (b): (c): (d): (e)] im Bereich von [(10 bis 1.000): (0,01 bis 1): (0,015 bis 1,5) : (0,013 bis 1,3): (0,005 bis 0,8)], insbesondere [(100 bis 300): (0,05 bis 0,5): (0,06 bis 0,6) : (0,055 bis 0,55): (0,02 bis 0,3)], aufweist, und/oder
wobei die Zusammensetzung ein Verhältnis (b) Pfefferminzöl zu (c) Anisöl zu (d) Fenchelöl zu (e) Kümmelöl [(b): (c): (d): (e)] im Bereich von [(0,01 bis 1): (0,015 bis 1,5): (0,013 bis 1,3): (0,005 bis 0,8)], insbesondere [(0,05 bis 0,5) : (0,06 bis 0,6) : (0,055 bis 0,55): (0,02 bis 0,3)], aufweist.

9. Zusammensetzung nach einem der vorangehenden Ansprüche,
wobei die Zusammensetzung außerdem mindestens einen pharmazeutischen Träger enthält, und/oder
wobei die Zusammensetzung in peroral verabreichbarer Applikationsform vorliegt, und/oder
wobei die Zusammensetzung flüssig und/oder viskos ist und/oder wobei die Zusammensetzung als Dispersion, insbesondere Suspension und/oder Emulsion, vorzugsweise Emulsion, vorliegt, und/oder
wobei die Zusammensetzung als flüssig basierte Dosiereinheit vorliegt, insbesondere wobei die Zusammensetzung als Dosiereinheit auf Basis einer Suspension, bevorzugt in Form von Dosiereinheiten für die einmalige Applikation, vorliegt, und/oder
wobei die Zusammensetzung in Dosiereinheiten auf Basis von jeweils 0,05 bis 5 g, insbesondere 0,1 bis 4 g, vorzugsweise 0,2 bis 2 g, der Zusammensetzung vorliegt.

10. Zusammensetzung nach einem der vorangehenden Ansprüche,
wobei die Zusammensetzung in ein Hüllmaterial und/oder Kapselmaterial, insbesondere Weichkapselmaterial, eingebracht vorliegt, insbesondere wobei das Hüllmaterial und/oder Kapselmaterial magensaftlöslich ausgebildet ist und/oder insbesondere wobei Hüllmaterial und/oder Kapselmaterial auf Basis von Gelatine ausgebildet ist und/oder wobei das Hüllmaterial und/oder Kapselmaterial insbesondere modifizierte Maisstärke, mindestens einen mehrwertigen Alkohol, insbesondere Glycerol, und/oder mindestens ein Polysaccharid, insbesondere Carrageenan, aufweist, und/oder
wobei die Zusammensetzung als Arzneimittel oder Pharmazeutikum, als Medizinprodukt, als Homöopathikum oder als Nahrungsergänzungsmittel, Nahrungsmittelzusatz oder Diätetikum ausgebildet ist.

11. Zusammensetzung, insbesondere pharmazeutische Zubereitung, vorzugsweise zur Verwendung zur therapeutischen oder prophylaktischen Behandlung von Verdauungsbeschwerden oder -störungen, insbesondere Verdauungsfehlfunktionen, Meteorismus, Flatulenz, Koliken, Völlegefühl, Magendruck, übermäßiger Gasbildung im Magen/Darm-Trakt oder dergleichen, insbesondere nach einem der vorangehenden Ansprüche,
wobei die Zusammensetzung - in Kombination und jeweils in wirksamen Mengen, insbesondere pharmazeutisch wirksamen Mengen -
(a) mindestens ein Polysiloxan in einer Menge von 60 bis 99,9 Gew.-%, besonders bevorzugt 70 bis 99,85 Gew.-%;
(b) Pfefferminzöl in einer Menge von 0,001 bis 1 Gew.-%, insbesondere 0,005 bis 0,5 Gew.-%, vorzugsweise 0,01 bis 0,1 Gew.-%, bevorzugt 0,015 bis 0,08 Gew.-%, besonders bevorzugt 0,02 bis 0,06 Gew.-%;
(c) Anisöl in Form von Sternanisöl in einer Menge von 0,0015 bis 1,5 Gew.-%, insbesondere 0,006 bis 1 Gew.-%, vorzugsweise 0,015 bis 0,15 Gew.-%, bevorzugt 0,02 bis 0,1 Gew.-%, besonders bevorzugt 0,025 bis 0,08 Gew.-%;
(d) Fenchelöl in Form von Bitterfenchelöl in einer Menge von 0,002 bis 1,3 Gew.-%, insbesondere 0,006 bis 0,7 Gew.-%, vorzugsweise 0,015 bis 0,12 Gew.-%, bevorzugt 0,02 bis 0,1 Gew.-%, besonders bevorzugt 0,025 bis 0,08 Gew.-%;
(e) Kümmelöl in einer Menge von 0,0005 bis 0,8 Gew.-%, insbesondere 0,001 bis 0,4 Gew.-%, vorzugsweise 0,005 bis 0,1 Gew.-%, bevorzugt 0,01 bis 0,06 Gew.-%, besonders bevorzugt 0,015 bis 0,05 Gew.-%,
jeweils bezogen auf die Zusammensetzung, enthält,
wobei die Zusammensetzung ein Verhältnis (a) Polysiloxan zu (b) Pfefferminzöl zu (c) Anisöl zu (d) Fenchelöl zu (e) Kümmelöl [(a) : (b): (c): (d): (e)] im Bereich von [(10 bis 1.000) : (0,01 bis 1) : (0,015 bis 1,5): (0,013 bis 1,3) : (0,005 bis 0,8)], insbesondere [(100 bis 300): (0,05 bis 0,5) : (0,06 bis 0,6): (0,055 bis 0,55): (0,02 bis 0,3)], aufweist und
wobei die Zusammensetzung enzymfrei ausgebildet ist.

12. Dosiereinheit, insbesondere zur peroralen Applikation, insbesondere in Form einer Kapsel, insbesondere Weichkapsel, enthaltend eine Zusammensetzung nach einem der Ansprüche 1 bis 11, und/oder insbesondere in Form einer vorzugsweise dosierfertigen Umverpackung, insbesondere Portionsbeutel, für die einmalige Applikation, enthaltend eine Zusammensetzung nach einem der Ansprüche 1 bis 11.

13. Verpackungseinheit, enthaltend mindestens eine Dosiereinheit, vorzugsweise eine Vielzahl von Dosiereinheiten, nach Anspruch 12.

14. Verwendung einer Zusammensetzung nach einem der Ansprüche 1 bis 11 zur Herstellung eines Arzneimittels zur therapeutischen oder prophylaktischen Behandlung von Verdauungsbeschwerden oder -störungen, insbesondere Verdauungsfehlfunktionen, Meteorismus, Flatulenz, Koliken, Völlegefühl, Magendruck, übermäßiger Gasbildung im Magen/Darm-Trakt oder dergleichen und/oder zur Herstellung eines Arzneimittels zur Verwendung als Karminativum und/oder zur Herstellung eines Arzneimittels mit karminativer Wirkung.

15. Karminativum, umfassend eine Zusammensetzung nach einem der Ansprüche 1 bis 11 und/oder umfassend eine Dosiereinheit nach Anspruch 12 und/oder umfassend eine Verpackungseinheit nach Anspruch 13.

## Claims

1. A composition, in particular a pharmaceutical preparation, preferably for use in the therapeutic or prophylactic treatment of digestive complaints or disorders, in particular digestive dysfunctions, meteorism, flatulence, colics, indigestion, stomach pressure, excessive gas formation in the gastrointestinal tract, or the like,
wherein the composition - in combination, and each in effective amounts, in particular in pharmaceutically effective amounts - comprises
(a) at least one polysiloxane in an amount of 60 to 99.9 wt.-%, based on the composition;
(b) peppermint oil in an amount of 0.001 to 1 wt.-%, based on the composition;
(c) anise oil in the form of star anise oil,
and in an amount of 0.0015 to 1.5 wt.-%, based on the composition;
(d) fennel oil in the form of bitter fennel oil,
and in an amount of 0.002 to 1.3 wt.-%, based on the composition;
(e) caraway oil in an amount of 0.0005 to 0.8 wt.-%, based on the composition;
wherein the composition is embodied to be free of any enzymes.

2. The composition according to claim 1, wherein (a) the polysiloxane is a polysiloxane having a surface energy modifying and/or defoaming effect, and/or wherein (a) the polysiloxane is preferably a linear alkyl or aryl polysiloxane, and/or wherein (a) the polysiloxane is Simeticon and/or Dimeticon, preferably Simeticon.

3. The composition according to claims 1 or 2,
comprising (a) the at least one polysiloxane in an amount of 70 to 99.85 wt.-%, based on the composition, and/or
comprising (a) the at least one polysiloxane per application and/or dosage unit in an amount of 10 to 1,000 mg, in particular 30 to 800 mg, preferably 50 to 600 mg, more preferred 80 to 400 mg, particularly preferred 100 to 350 mg, and/or
comprising (a) the at least one polysiloxane per application and/or dosage unit in an amount of at least 10 mg, in particular at least 30 mg, preferably at least 50 mg, more preferred at least 80 mg, particularly preferred at least 100 mg, and/or comprising (a) the at least one polysiloxane per application and/or dosage unit in an amount of not more than 1,000 mg, in particular not more than 800 mg, preferably not more than 600 mg, more preferred not more than 400 mg, particularly preferred not more than 350 mg,
and/or
comprising (a) the at least one polysiloxane per application and/or dosage unit in an amount of at least 10 mg, in particular at least 30 mg, preferably at least 50 mg, more preferred at least 80 mg, particularly preferred at least 100 mg, and/or comprising (a) the at least one polysiloxane per application and/or dosage unit in an amount of not more than 1,000 mg, in particular not more than 800 mg, preferably not more than 600 mg, more preferred not more than 400 mg, particularly preferred not more than 350 mg.

4. The composition according to one of the previous claims,
comprising (b) the peppermint oil in an amount of 0.005 to 0.5 wt.-%, preferably 0.01 to 0.1 wt.-%, more preferred 0.015 to 0.08 wt.-%, particularly preferred 0.02 to 0.06 wt.-%, based on the composition, and/or
comprising (b) the peppermint oil in an amount of at least 0.001 wt.-%, in particular at least 0.005 wt.-%, preferably at least 0.01 wt.-%, preferably at least 0.015 wt.-%, particularly preferred at least 0.02 wt.-%, based on the composition, and/or comprising (b) the peppermint oil in an amount of not more than 1 wt.-%, in particular not more than 0.5 wt.-%, preferably not more than 0.1 wt.-%, more preferably not more than 0.08 wt.-%, particularly preferred not more than 0.06 wt.-%, based on the composition, and/or
comprising (b) the peppermint oil in an amount of 0.001 to 1 mg, in particular 0.01 to 0.8 mg, preferably 0.02 to 0.6 mg, more preferably 0.05 to 0.5 mg, particularly preferred 0.06 to 0.25 mg.

5. The composition according to one of the previous claims,
comprising (c) the anise oil in an amount of 0.006 to 1 wt.-%, preferably 0.015 to 0.15 wt.-%, preferably 0.02 to 0.1 wt.-%, particularly preferred 0.025 to 0.08 wt.-%, based on the composition, and/or
comprising (c) the anise oil in an amount of at least 0.0015 wt.-%, in particular at least 0.006 wt.-%, preferably at least 0.015 wt.-%, more preferred at least 0.02 wt.-%, particularly preferred at least 0.025 wt.-%, based on the composition, and/or comprising (c) the anise oil in an amount of not more than 1.5 wt.-%, in particular not more than 1 wt.-%, preferably not more than 0.15 wt.-%, more preferably not more than 0.1 wt.-%, particularly preferred not more than 0.08 wt.-%, based on the composition, and/or
comprising (c) the anise oil per application and/or dosage unit in an amount of 0.002 to 1.5 mg, in particular 0.02 to 1.2 mg, preferably 0.04 to 1 mg, more preferred 0.05 to 0.8 mg, particularly preferred 0.06 to 0.3 mg.

6. The composition according to one of the previous claims,
comprising (d) the fennel oil in an amount of 0.006 to 0.7 wt.-%, preferably 0.015 to 0.12 wt.-%, more preferably 0.02 to 0.1 wt.-%, particularly preferred 0.025 to 0.08 wt.-%, based on the composition, and/or
comprising (d) the fennel oil in an amount of at least 0.002 wt.-%, in particular at least 0.006 wt.-%, preferably at least 0.015 wt.-%, more preferred at least 0.02 wt.-%, particularly preferred 0.025 wt.-%, based on the composition, and/or comprising (d) the fennel oil in an amount of not more than 1.3 wt.-%, in particular not more than 0.7 wt.-%, preferably not more than 0.12 wt.-%, more preferred not more than 0.1 wt.-%, particularly preferred 0.08 wt.-%, based on the composition, and/or
comprising (d) the fennel oil per application and/or dosage unit in an amount of 0.0015 to 1.3 mg, in particular 0.015 to 1 mg, preferably 0.03 to 0.8 mg, more preferably 0.05 to 0.7 mg, particularly preferred 0.06 to 0.25 mg.

7. The composition according to one of the previous claims,
comprising (e) the caraway oil in an amount of 0.001 to 0.4 wt.-%, preferably 0.005 to 0.1 wt.-%, more preferred 0.01 to 0.06 wt.-%, particularly preferred 0.015 to 0.05 wt.-%, based on the composition, and/or
comprising (e) the caraway oil in an amount of at least 0.0005 wt.-%, in particular at least 0.001 wt.-%, preferably at least 0.005 wt.-%, more preferably at least 0.01 wt.-%,
particularly preferred at least 0.015 wt.-%, based on the composition, and/or comprising (e) the caraway oil in an amount of not more than 0.8 wt.-%, in particular not more than 0.4 wt.-%, preferably not more than 0.1 wt.-%, more preferably not more than 0.06 wt.-%, particularly preferred not more than 0.05 wt.-%, based on the composition, and/or
comprising (e) the caraway oil per application and/or dosage unit in an amount of 0.001 to 0.8 mg, in particular 0.005 to 0.6 mg, preferably 0.01 to 0.5 mg, more preferably 0.02 to 0.2 mg, particularly preferred 0.03 to 0.1 mg.

8. The composition according to one of the previous claims,
wherein the composition has a ratio of (a) polysiloxane to (b) peppermint oil, and (c) anise oil, and (d) fennel oil, and (e) caraway oil [(a) : ((b) + (c) + (d) + (e))] in the range of [(10 to 1,000) : (0.1 to 5)], in particular [(100 to 300) : (0.2 to 2)], and/or
wherein the composition has a ratio of (a) polysiloxane to (b) peppermint oil to (c) anise oil to (d) fennel oil to (e) caraway oil [(a) : (b) : (c) : (d) : (e)] in the range of [(10 to 1,000)
: (0.01 to 1) : (0.015 to 1.5) : (0.013 to 1.3) : (0.005 to 0.8)], in particular of [(100 to 300) : (0.05 to 0.5) : (0.06 to 0.6) : (0.055 to 0.55) : (0.02 to 0.3)], and/or
wherein the composition has a ratio of (b) peppermint oil to (c) anise oil to (d) fennel oil to (e) caraway oil [(b) : (c) : (d) : (e)] in the range of [(0.01 to 1) : (0.015 to 1.5) : (0.013 to 1.3) : (0.005 to 0.8)], in particular [(0.05 to 0.5) : (0.06 to 0.6) : (0.055 to 0.55) : (0.02 to 0.3)].

9. The composition according to one of the previous claims,
wherein the composition further has at least one pharmaceutical carrier, and/or wherein the composition is present in an application form that may be administered perorally, and/or
wherein the composition is present is liquid and/or viscous, and/or wherein the composition is present as a dispersion, in particular a suspension and/or emulsion, preferably an emulsion, and/or
wherein the composition is present as a dosage unit based on a liquid, in particular wherein the composition is present as a dosage unit based on a suspension, preferably in the form of dosage units for one-time use applications, and/or
wherein the composition is present in dosage units based on 0.05 to 5 g each, in particular 0.1 to 4 g each, preferably 0.2 to 2 g each, of the composition.

10. The composition according to one of the previous claims,
wherein the composition is incorporated in a shell material and/or capsule material, in particular soft capsule material, in particular, wherein the shell material and or the capsule material is embodied in a gastric juice soluble manner, and/or in particular, wherein the shell material and/or the capsule material is embodied based on gelatin, and/or wherein the shell material and/or the capsule material is, in particular, modified corn starch, at least one polyvalent alcohol, in particular glycerol, and/or at least one polysaccharide, in particular carrageenan, and/or
wherein the composition is embodied as a medicament or pharmaceutical, as a medical product as a homeopathic remedy, or as a nutritional supplement, a food additive, or a dietetic.

11. The composition, in particular a pharmaceutical preparation, preferably for use in the therapeutic or prophylactic treatment of digestive complaints or disorders, in particular digestive dysfunctions, meteorism, flatulence, colics, indigestion, stomach pressure, excessive gas formation in the gastrointestinal tract, or the like, in particular according to one of the previous claims,
wherein the composition - in combination, and each in effective amounts, in particular in pharmaceutically effective amounts - comprises
(a) at least one polysiloxane in an amount of 60 to 99.9 wt.-%, particularly preferred 70 to 99.85 wt.-%;
(b) peppermint oil in an amount of 0.001 to 1 wt.-%, in particular 0.005 to 0.5 wt.-%, preferably 0.01 to 0.1 wt.-%, more preferred 0.015 to 0.08 wt.-%, particularly preferred 0.02 to 0.06 wt.-%;
(c) anise oil in the form of star anise oil, in an amount of 0.0015 to 1.5 wt.-%, in particular 0.006 to 1 wt.-%, preferably 0.015 to 0.15 wt.-%, more preferred 0.02 to 0.1 wt.-%, particularly preferred 0.025 to 0.08 wt.-%;
(d) fennel oil in the form of bitter fennel oil in an amount of 0.002 to 1.3 wt.-%, in particular 0.006 to 0.7 wt.-%, preferably 0.015 to 0.12 wt.-%, more preferred 0.02 to 0.1 wt.-%, particularly preferred 0.025 to 0.08 wt.-%;
(e) caraway oil in an amount of 0.0005 to 0.8 wt.-%, in particular 0.001 to 0.4 wt.-%, preferably 0.005 to 0.1 wt.-%, more preferably 0.01 to 0.06 wt.-%, particularly preferred 0.015 to 0.05 wt.-%,
each based on the composition,
wherein the composition has a ratio of wherein the composition has a ratio of (a) polysiloxane to (b) peppermint oil to (c) anise oil to (d) fennel oil to (e) caraway oil [(a) : (b) : (c) : (d) : (e)] in the range of [(10 to 1,000) : (0.01 to 1) : (0.015 to 1.5) : (0.013 to 1.3) : (0.005 to 0.8)], in particular of [(100 to 300) : (0.05 to 0.5) : (0.06 to 0.6) : (0.055 to 0.55) : (0.02 to 0.3)], and/or
wherein the composition is embodied to be free of any enzymes.

12. A dosage unit, in particular for peroral application, in particular in the form of a capsule, in particular a soft capsule, comprising a composition according to one of the claims 1 to 11, and/or in particular in the form of a preferably dosage-ready secondary packaging, in particular a sealed packet, for one-time application, comprising a composition according to one of the claims 1 to 11.

13. A packaging unit, comprising at least one dosage unit, preferably a plurality of dosage units, according to claim 12.

14. A use of a composition according to one of the claims 1 to 11 for the production of a medicament for the therapeutic or prophylactic treatment of digestive complaints or disorders, in particular digestive dysfunctions, meteorism, flatulence, colics, indigestion, stomach pressure, excessive gas formation in the gastrointestinal tract, or the like, and/or the production of a medicament for use as a carminative, and/or for the production of a medicament having a carminative effect.

15. A carminative, comprising a composition according to one of the claims 1 to 11, and/or comprising a dosage unit according to claim 12, and/or comprising a packaging unit according to claim 13.

## Revendications

1. Composition, en particulier préparation pharmaceutique, de préférence pour utilisation pour le traitement thérapeutique ou prophylactique de douleurs ou de troubles de la digestion, en particulier des dysfonctionnements digestifs, du météorisme, des flatulences, des coliques, de la sensation de plénitude, de la pression gastrique, une formation excessive de gaz dans le tractus gastro-intestinal ou analogues,
la composition - en combinaison et dans chaque cas en des quantités efficaces, en particulier en des quantités pharmaceutiquement efficaces - contient
(a) au moins un polysiloxane en une quantité de 60 à 99,9 % en poids par rapport à la composition ;
(b) de l'essence de menthe poivrée en une quantité de 0,001 à 1 % en poids par rapport à la composition ;
(c) de l'essence d'anis sous forme d'essence d'anis étoilé en une quantité de 0,0015 à 1,5 % en poids par rapport à la composition ;
(d) de l'essence de fenouil sous forme d'essence de fenouil amer en une quantité de 0,002 à 1,3 % en poids par rapport à la composition ;
(e) de l'essence de carvi en une quantité de 0,0005 à 0,8 % en poids par rapport à la composition,
la composition étant réalisée sans enzyme.

2. Composition selon la revendication 1, dans laquelle (a) le polysiloxane est un polysiloxane ayant un effet modifiant la tension superficielle et/ou antimoussant, et/ou dans laquelle (a) le polysiloxane est un alkyl- ou un arylpolysiloxane de préférence linéaire, et/ou dans lequel (a) le polysiloxane est la siméthicone et/ou la diméthicone, de préférence la siméthicone.

3. Composition selon la revendication 1 ou 2,
contenant (a) le ou les polysiloxanes en une quantité de 70 à 99,85 % en poids par rapport à la composition, et/ou
contenant (a) le ou les polysiloxanes, par unité d'administration et/ou par unité posologique, en une quantité de 10 à 1000 mg, en particulier de 30 à 800 mg, de préférence de 50 à 600 mg, préférentiellement de 80 à 400 mg, d'une manière particulièrement préférée de 100 à 350 mg, et/ou
contenant (a) le ou les polysiloxanes, par unité d'administration et/ou par unité posologique, en une quantité d'au moins 10 mg, en particulier d'au moins 30 mg, de préférence d'au moins 50 mg, préférentiellement d'au moins 80 mg, d'une manière particulièrement préférée d'au moins 100 mg, et/ou contenant (a) le ou les polysiloxanes, par unité d'administration et/ou par unité posologique, en une quantité d'au plus 1000 mg, en particulier d'au plus 800 mg, de préférence d'au plus 600 mg, préférentiellement d'au plus 400 mg, d'une manière particulièrement préférée d'au plus 350 mg, et/ou
contenant (a) le ou les polysiloxanes, par unité d'administration et/ou par unité posologique, en une quantité d'au moins 10 mg, en particulier d'au moins 30 mg, de préférence d'au moins 50 mg, préférentiellement d'au moins 80 mg, d'une manière particulièrement préférée d'au moins 100 mg, et/ou contenant (a) le ou les polysiloxanes, par unité d'administration et/ou par unité posologique, en une quantité d'au plus 1000 mg, en particulier d'au plus 800 mg, de préférence d'au plus 600 mg, préférentiellement d'au plus 400 mg, d'une manière particulièrement préférée d'au plus 350 mg.

4. Composition selon l'une des revendications précédentes,
contenant (b) l'essence de menthe poivrée en une quantité de 0,005 à 0,5 % en poids, de préférence de 0,01 à 0,1 % en poids, préférentiellement de 0,015 à 0,08 % en poids, d'une manière particulièrement préférée de 0,02 à 0,06 % en poids, par rapport à la composition, et/ou
contenant (b) l'essence de menthe poivrée en une quantité d'au moins 0,001 % en poids, en particulier d'au moins 0,005 % en poids, de préférence d'au moins 0,01 % en poids, préférentiellement d'au moins 0,015 % en poids, d'une manière particulièrement préférée d'au moins 0,02 % en poids, par rapport à la composition, et/ou contenant (b) l'essence de menthe poivrée en une quantité d'au plus 1 % en poids, en particulier d'au plus 0,5 % en poids, de préférence d'au plus 0,1 % en poids, préférentiellement d'au plus 0,08% en poids, d'une manière particulièrement préférée d'au plus 0,06 % en poids, par rapport à la composition, et/ou
contenant (b) l'essence de menthe poivrée, par unité d'administration et/ou par unité posologique, en une quantité de 0,001 à 1 mg, en particulier de 0,01 à 0,8 mg, de préférence de 0,02 à 0,6 mg, préférentiellement de 0,05 à 0,5 mg, d'une manière particulièrement préférée de 0,06 à 0,25 mg.

5. Composition selon l'une des revendications précédentes,
contenant (c) l'essence d'anis en une quantité de 0,006 à 1 % en poids, de préférence de 0,015 à 0,15 % en poids, préférentiellement de 0,02 à 0,1 % en poids, d'une manière particulièrement préférée de 0,025 à 0,08 % en poids, par rapport à la composition, et/ou
contenant (c) l'essence d'anis en une quantité d'au moins 0,0015 % en poids, en particulier d'au moins 0,006 % en poids, de préférence d'au moins 0,015 % en poids, préférentiellement d'au moins 0,02 % en poids, d'une manière particulièrement préférée d'au moins 0,025 % en poids, par rapport à la composition, et/ou contenant (c) l'essence d'anis en une quantité d'au plus 1,5 % en poids, en particulier d'au plus 1 % en poids, de préférence d'au plus 0,15 % en poids, préférentiellement d'au plus 0,1 % en poids, d'une manière particulièrement préférée d'au plus 0,08 % en poids, par rapport à la composition, et/ou
contenant (c) l'essence d'anis, par unité d'administration et/ou par unité posologique, en une quantité de 0,002 à 1,5 mg, en particulier de 0,02 à 1,2 mg, de préférence de 0,04 à 1 mg, préférentiellement de 0,05 à 0,8 mg, d'une manière particulièrement préférée de 0,06 à 0,3 mg.

6. Composition selon l'une des revendications précédentes,
contenant (d) l'essence de fenouil en une quantité de 0,006 à 0,7 % en poids, de préférence de 0,015 à 0,12 % en poids, préférentiellement de 0,02 à 0,1 % en poids, d'une manière particulièrement préférée de 0,025 à 0,08 % en poids, par rapport à la composition, et/ou
contenant (d) l'essence de fenouil en une quantité d'au moins 0,002 % en poids, en particulier d'au moins 0,006 % en poids, de préférence d'au moins 0,015 % en poids, préférentiellement d'au moins 0,02 % en poids, d'une manière particulièrement préférée de 0,025 % en poids, par rapport à la composition, et/ou contenant (d) l'essence de fenouil en une quantité d'au plus 1,3 % en poids, en particulier d'au plus 0,7 % en poids, de préférence d'au plus 0,12 % en poids, préférentiellement d'au plus 0,1 % en poids, d'une manière particulièrement préférée d'au plus 0,08 % en poids, par rapport à la composition, et/ou
contenant (d) l'essence de fenouil, par unité d'administration et/ou par unité posologique, en une quantité de 0,0015 à 1,3 mg, en particulier de 0,015 à 1 mg, de préférence de 0,03 à 0,8 mg, préférentiellement de 0,05 à 0,7 mg, d'une manière particulièrement préférée de 0,06 à 0,25 mg.

7. Composition selon l'une des revendications précédentes contenant (e) l'essence de carvi en une quantité de 0,001 à 0,4 % en poids, de préférence de 0,005 à 0,1 % en poids, préférentiellement de 0,01 à 0,06 % en poids, d'une manière particulièrement préférée de 0,015 à 0,05 % en poids, par rapport à la composition, et/ou
contenant (e) l'essence de carvi en une quantité d'au moins 0,0005 % en poids, en particulier d'au moins 0,001 % en poids, de préférence d'au moins 0,005 % en poids, préférentiellement d'au moins 0,01 % en poids, d'une manière particulièrement préférée d'au moins 0,015 % en poids, par rapport à la composition, et/ou contenant (e) l'essence de carvi en une quantité d'au plus 0,8 % en poids, en particulier d'au plus 0,4 % en poids, de préférence d'au plus 0,1 % en poids, préférentiellement d'au plus 0,06 % en poids, d'une manière particulièrement préférée d'au plus 0,05 % en poids, par rapport à la composition, et/ou
contenant (e) l'essence de carvi, par unité d'administration et/ou par unité posologique, en une quantité de 0,001 à 0,8 mg, en particulier de 0,005 à 0,6 mg, de préférence de 0,01 à 0,5 mg, préférentiellement de 0,02 à 0,2 mg, d'une manière particulièrement préférée de 0,03 à 0,1 mg.

8. Composition selon l'une des revendications précédentes,
la composition présentant un rapport entre (a) le polysiloxane d'une part, et (b) l'essence de menthe poivrée et (c) l'essence d'anis et (d) l'essence de fenouil et (e) l'essence de carvi d'autre part [(a) : ((b) + (c) + (d) + (e))], compris dans la plage de [(10 à 1000) : (0,1 à 5)], en particulier de [(100 à 300) : (0,2 à 2)], et/ou
la composition présentant une proportion (a) polysiloxane : (b) essence de menthe poivrée : (c) essence d'anis : (d) essence de fenouil : (e) essence de carvi [(a) : (b) : (c) : (d) : (e)] comprise dans la plage de [(10 à 1000) : (0,01 à 1) : (0,015 à 1,5) : (0,013 à 1,3) : (0, 005 à 0,8)], en particulier de [(100 à 300) : (0,05 à 0,5) : (0,06 à 0,6) : (0,055 à 0,55) : (0,02 à 0,3)], et/ou
la composition présentant une proportion (b) essence de menthe poivrée : (c) essence d'anis : (d) essence de fenouil : (e) essence de carvi [(b) : (c) : (d) : (e)] comprise dans la plage de [(0,01 à 1) : (0,015 à 1,5) : (0,013 à 1,3) : (0,005 à 0,8)], en particulier de [(0,05 à 0,5) : (0,06 à 0,6) : (0,055 à 0,55) : (0,02 à 0,3)].

9. Composition selon l'une des revendications précédentes,
la composition contenant en outre au moins un support pharmaceutique, et/ou
la composition se présentant sous une forme d'administration pouvant être administrée par voie orale, et/ou
la composition étant liquide et/ou visqueuse, et/ou la composition se présentant sous forme d'une dispersion, en particulier d'une suspension et/ou d'une émulsion, de préférence d'une émulsion, et/ou
la composition se présentant sous forme d'une unité posologique à base liquide, en particulier la composition se présentant sous forme d'une unité posologique à base d'une suspension, de préférence sous forme d'unités posologiques pour administration unique, et/ou
la composition se présentant dans des unités posologiques dont chacune est à base de 0,05 à 5 g, en particulier de 0,1 à 4 g, de préférence de 0,2 à 2 g de la composition.

10. Composition selon l'une des revendications précédentes
la composition se présentant mise en place dans un matériau d'enveloppe et/ou un matériau pour capsule, en particulier un matériau pour capsule molle, en particulier le matériau d'enveloppe et/ou le matériau pour capsule ayant une constitution gastrosoluble, et en particulier le matériau d'enveloppe et/ou le matériau pour capsule étant réalisés à base de gélatine et/ou le matériau d'enveloppe et/ou le matériau pour capsule comprenant en particulier de l'amidon de maïs modifié, au moins un polyalcool, en particulier le glycérol, et/ou au moins un polysaccharide, en particulier le carragénane,
la composition étant réalisée sous forme d'un médicament ou d'un produit pharmaceutique, d'un produit à usage médical, d'un produit homéopathique, ou sous forme d'un complément alimentaire, d'un additif alimentaire ou d'un produit diététique.

11. Composition, en particulier préparation pharmaceutique, de préférence pour utilisation pour le traitement thérapeutique ou prophylactique de douleurs ou de troubles de la digestion, en particulier des dysfonctionnements digestifs, du météorisme, des flatulences, des coliques, de la sensation de plénitude, de la pression gastrique, de la formation excessive de gaz dans le tractus gastro-intestinal ou analogues, en particulier selon l'une des revendications précédentes,
la composition - en combinaison et dans chaque cas en des quantités efficaces, en particulier en des quantités pharmaceutiquement efficaces - contenant
(a) au moins un polysiloxane en une quantité de 60 à 99,9 % en poids, d'une manière particulièrement préférée de 70 à 99,85 % en poids ;
(b) de l'essence de menthe poivrée en une quantité de 0,001 à 1 % en poids, en particulier de 0,005 à 0,5 % en poids, de préférence de 0,01 à 0,1 % en poids, préférentiellement de 0,015 à 0,08 % en poids, d'une manière particulièrement préférée de 0,02 à 0,06 % en poids ;
(c) de l'essence d'anis sous forme d'essence d'anis étoilé, en une quantité de 0,0015 à 1,5 % en poids, en particulier de 0,006 à 1 % en poids, de préférence de 0,015 à 0,15 % en poids, préférentiellement de 0,02 à 0,1 % en poids, d'une manière particulièrement préférée de 0,025 à 0,08 % en poids ;
(d) de l'essence de fenouil sous forme d'essence de fenouil amer, en une quantité de 0,002 à 1,3 % en poids, en particulier de 0,006 à 0,7 % en poids, de préférence de 0,015 à 0,12 % en poids, préférentiellement de 0,02 à 0,1 % en poids, d'une manière particulièrement préférée de 0,025 à 0,08 % en poids ;
(e) de l'essence de carvi en une quantité de 0,0005 à 0,8 % en poids, en particulier de 0,001 à 0,4 % en poids, de préférence de 0,005 à 0,1 % en poids, préférentiellement de 0,01 à 0,06 % en poids, d'une manière particulièrement préférée de 0,015 à 0,05 % en poids,
dans chaque cas par rapport à la composition,
la composition présentant une proportion (a) polysiloxane : (b) essence de menthe poivrée : (c) essence d'anis : (d) essence de fenouil : (e) essence de carvi [(a) : (b) : (c) : (d) : (e)] comprise dans la plage de [(10 à 1000) : (0,01 à 1) : (0,015 à 1,5) : (0,013 à 1,3) : (0,005 à 0,8)], en particulier de [(100 à 300) : (0,05 à 0,5) : (0,06 à 0,6) : (0,055 à 0,55) : (0, 02 à 0,3)],
la composition étant réalisée sans enzyme.

12. Unité posologique, en particulier pour administration par voie orale, en particulier sous forme d'une capsule, en particulier d'une capsule molle, contenant une composition selon l'une des revendications 1 à 11, et/ou en particulier sous forme d'un reconditionnement de préférence prêt au dosage, en particulier d'un sachet portion, pour une administration unique, contenant une composition selon l'une des revendications 1 à 11.

13. Unité de conditionnement contenant au moins une unité posologique, de préférence un grand nombre d'unités posologiques, selon la revendication 12.

14. Utilisation d'une composition selon l'une des revendications 1 à 11 pour fabriquer un médicament pour le traitement thérapeutique ou prophylactique des douleurs ou troubles de la digestion, en particulier des dysfonctionnements digestifs, du météorisme, des flatulences, des coliques, de la sensation de plénitude, de la pression gastrique, de la formation excessive de gaz dans le tractus gastro-intestinal ou analogues, et/ou pour la fabrication d'un médicament pour utilisation en tant que carminatif et/ou pour utilisation d'un médicament à effet carminatif.

15. Carminatif comprenant une composition selon l'une des revendications 1 à 11 et/ou comprenant une unité posologique selon la revendication 12 et/ou comprenant une unité de conditionnement selon la revendication 13.
